# EUROPEAN PATENT APPLICATION

(11) **EP 4 223 266 A1**
(43) Date of publication of application: **09.08.2023**
(21) Application number: 21875726.8
(22) Date of filing: 29.09.2021
(51) Int. Cl.: A61F 13/15, A61F 13/53

(54) **ABSORBENT ARTICLE AND METHOD OF MANUFACTURING CORE WRAP SHEET**

(30) Priority: 30.09.2020 JP 2020165888
(71) Applicant: Unicharm Corporation, Shikokuchuo-shi, Ehime 799-0111 (JP)
(72) Inventor: ONISHI, Kazuaki, Kanonji-shi, Kagawa 769-1602 (JP)
(74) Representative: Staeger & Sperling Partnerschaftsgesellschaft mbB
(86) International application number: PCT/JP2021/035956
(87) International publication number: WO 2022/071429

(57) **Abstract**

The present invention provides an absorbent article which is capable of maintaining a higher level liquid absorbency even when repeatedly absorbing a bodily fluid such as urine and a method of manufacturing a core wrap sheet. An absorbent 7A which has a skin-facing surface 15 and a non-skin-facing surface 17 and which comprises an absorbent core 9A and a core wrap sheet 11A disposed on the skin-facing surface 15 side and/or on the non-skin-facing surface 17 side. The core wrap sheet 11A comprises an inner layer 16A and an outer layer 18A. The inner layer 16A contains 50 mass% or more of a cellulose fiber and comprises a core-facing surface 21 being in contact with the absorbent core 9A a non-core facing surface 23 and a plurality of open holes 27. The outer layer 18A contains 50 mass% or more of a thermoplastic synthetic fiber and comprises a first surface 29 being in contact with the non-core facing surface 23 a second surface 31 a plurality of first grooves 33 which are formed on the first surface 29 and recessed in the thickness direction and a plurality of second grooves 35 which are formed on the second surface 31 and recessed in the thickness direction.

## Description

### FIELD

The present invention relates to an absorbent article and a method of manufacturing a core-wrapping sheet.

### BACKGROUND

Conventionally, in an absorbent article such as a disposable diaper or a sanitary napkin, an absorbent body in which an absorbent core formed of hydrophilic fibers such as wood pulp fibers and superabsorbent polymer particles dispersed in the hydrophilic fibers is covered by a core-wrapping sheet is used.

Patent Literature 1 discloses an absorbent article including a liquid-permeable top sheet, a liquid-impermeable back sheet, and an absorbent body arranged between the top sheet and the back sheet. The absorbent body includes an absorbent core and a core-wrapping sheet that covers the lower surface. The core-wrapping sheet contains 50 mass% or more of thermoplastic synthetic fibers, and a plurality of groove portions and ridge portions are alternately located on the surface. The plurality of groove portions and the plurality of ridge portions extend in the lengthwise direction of the absorbent core and are alternately located with predetermined spaces in the width direction of the absorbent core.

### [CITATION LIST]

### [PATENT LITERATURE]

Patent Literature 1: Japanese Unexamined Patent Publication No. 2018-000525

### SUMMARY

### [TECHNICAL PROBLEM]

In a case of Patent Literature 1, since a restorable liquid transport channel is secured between the absorbent core and the core-wrapping sheet and between the core-wrapping sheet and the liquid-impermeable back sheet, the strength is less likely to decrease even in a case where a large amount of bodily fluid is absorbed, and high absorbency can be maintained. Since the maintenance of absorbency leads to improvement in feeling of wearing of an absorbent article, an absorbent article with enhanced liquid absorbency is desired.

An aspect of the present invention is to provide an absorbent article capable of maintaining liquid absorbency at a higher level even in a case where a bodily fluid such as urine is repeatedly absorbed, and a method of manufacturing a core-wrapping sheet.

### [SOLUTION TO PROBLEM]

The present invention provides an absorbent article comprising:
a liquid-permeable top sheet;
a liquid-impermeable back sheet; and
an absorbent body that is arranged between the top sheet and the back sheet, wherein
the absorbent body includes
   an absorbent core having a skin facing surface and a non-skin facing surface on a side opposite to the skin facing surface, and having a lengthwise direction, a width direction, and a thickness direction, and
   a core-wrapping sheet that is arranged at least one side of the skin facing surface and the non-skin facing surface,
the core-wrapping sheet has an inner layer and an outer layer,
the inner layer contains 50 mass% or more of cellulose fibers, and has a core facing surface in contact with the absorbent core, a non-core facing surface on a side opposite to the core facing surface, and a plurality of open holes penetrating the inner layer from the core facing surface to the non-core facing surface,
the outer layer contains 50 mass% or more of thermoplastic synthetic fibers and has a first surface in contact with the non-core facing surface, a second surface on a side opposite to the first surface, a plurality of first grooves recessed in the thickness direction on the first surface, and a plurality of second grooves recessed in the thickness direction on the second surface, and
each of the plurality of first grooves and each of the plurality of second grooves are alternately arranged with predetermined spaces.

### [ADVANTAGEOUS EFFECT OF THE INVENTION]

The absorbent article according to the present invention can maintain liquid absorbency at a higher level even in a case where a bodily fluid such as urine is repeatedly absorbed.

### BRIEF DESCRIPTION OF DRAWINGS

FIG. 1 is a plan view of an absorbent article according to a first embodiment.
FIG. 2 is a cross-sectional view of an absorbent body according to the first embodiment taken along a line II-II in FIG. 1.
FIG. 3 is a partially enlarged cross-sectional view of the absorbent body according to the first embodiment.
FIG. 4 is a partial cross-sectional perspective view of a core-wrapping sheet according to the first embodiment.
FIG. 5 is a schematic view showing an example of a manufacturing apparatus for manufacturing the core-wrapping sheet according to the first embodiment.
FIG. 6 is a schematic view showing a shaping roll that can be used in the manufacturing apparatus for manufacturing the core-wrapping sheet according to the first embodiment.
FIG. 7A is a cross-sectional view of an absorbent body according to modified example (1) of the first embodiment taken along a line II-II in FIG. 1.
FIG. 7B is a cross-sectional view of an absorbent body according to modified example (2) of the first embodiment taken along a line II-II in FIG. 1.
FIG. 8 is a partially enlarged cross-sectional view of an absorbent body according to modified example (3) of the first embodiment.
FIG. 9 is a partial cross-sectional perspective view of a core-wrapping sheet according to a second embodiment.
FIG. 10 is a plan view of the core-wrapping sheet according to the second embodiment.
FIG. 11 is a schematic view showing a shaping roll that can be used in a manufacturing apparatus for manufacturing the core-wrapping sheet according to the second embodiment.
FIG. 12 is a plan view of an absorbent core of the absorbent article according to the third embodiment.
FIG. 13 is a cross-sectional view of an absorbent core according to a third embodiment taken along a line XIII-XIII in FIG. 12.
FIG. 14 is a cross-sectional view of an absorbent core according to a modified example of the third embodiment taken along a line XIII-XIII in FIG. 12.

### DESCRIPTION OF EMBODIMENTS

Embodiments of the present invention relate to the following aspects.

### [Aspect 1]

An absorbent article comprising:
a liquid-permeable top sheet;
a liquid-impermeable back sheet; and
an absorbent body that is arranged between the top sheet and the back sheet, wherein
the absorbent body includes
   an absorbent core having a skin facing surface and a non-skin facing surface on a side opposite to the skin facing surface, and having a lengthwise direction, a width direction, and a thickness direction, and
   a core-wrapping sheet that is arranged at least one side of the skin facing surface and the non-skin facing surface,
the core-wrapping sheet has an inner layer and an outer layer,
the inner layer contains 50 mass% or more of cellulose fibers, and has a core facing surface in contact with the absorbent core, a non-core facing surface on a side opposite to the core facing surface, and a plurality of open holes penetrating the inner layer from the core facing surface to the non-core facing surface,
the outer layer contains 50 mass% or more of thermoplastic synthetic fibers and has a first surface in contact with the non-core facing surface, a second surface on a side opposite to the first surface, a plurality of first grooves recessed in the thickness direction on the first surface, and a plurality of second grooves recessed in the thickness direction on the second surface, and
each of the plurality of first grooves and each of the plurality of second grooves are alternately arranged with predetermined spaces.

The bodily fluid excreted on the top sheet surface permeates the top sheet. In a case where the core-wrapping sheet covers the skin facing surface, the bodily fluid permeates the outer layer, reaches between the outer layer and the inner layer, and also reaches between the inner layer and the absorbent core through the plurality of open holes in the inner layer. Further, in a case where the core-wrapping sheet covers the non-skin facing surface, a part of the bodily fluid that has permeated the top sheet is absorbed by the absorbent core, and the bodily fluid that has not been absorbed by the absorbent core reaches between the absorbent core and the inner layer, and also reaches between the inner layer and the outer layer through the plurality of open holes in the inner layer. The space between the absorbent core and the inner layer will be referred to as a first diffusion space, and the space between the inner layer and the outer layer will be referred to as a second diffusion space. In any of the these cases, due to the diffusivity in the inner layer containing 50 mass% or more of cellulose fibers, the bodily fluid is diffused into the inside of the inner layer by the capillary phenomenon and also diffused through the first diffusion space and the second diffusion space, respectively. Further, the bodily fluid is diffused through a third diffusion space having the second grooves. Therefore, in the present absorbent article, in addition to the first diffusion space, the second diffusion space, and the third diffusion space, the capillary phenomenon that occurs inside the inner layer makes it possible to more efficiently diffuse the bodily fluid, and thus liquid absorbency can be maintained at a higher level. Since the outer layer contains 50 mass% or more of thermoplastic synthetic fibers, the strength is less likely to decrease even in a case where the bodily fluid is repeatedly absorbed, and thus deformation of the plurality of first grooves and the plurality of second grooves can be suppressed. Therefore, in the present absorbent article, a large amount of bodily fluid can be diffused through the plurality of first grooves and the plurality of second grooves even in a case where the bodily fluid is repeatedly absorbed.

### [Aspect 2]

The absorbent article according to aspect 1, wherein
the non-core facing surface and the first surface are not joined to each other.

In the present absorbent article, since the second diffusion space is more reliably formed, the bodily fluid can be more reliably diffused into the second diffusion space. Therefore, in the present absorbent article, the liquid absorbency can be maintained at a higher level. In a case where the non-core facing surface and the first surface are joined with an adhesive, and in a case where the non-core facing surface and the first surface are joined by entangling the fibers of the inner layer and the outer layer, the second diffusion space is blocked, and thus the bodily fluid can be interrupted from diffusing. In a case where the non-core facing surface and the first surface are joined by embossing, the bodily fluid remains in a portion in which the density of fibers is increased by the embossing and the bodily fluid can be interrupted from diffusing.

### [Aspect 3]

The absorbent article according to aspect 1 or 2, wherein
each of the plurality of first grooves and each of the plurality of second grooves extend in a direction parallel to the lengthwise direction, and are alternately arranged with predetermined spaces in a direction parallel to the width direction.

Since the bodily fluid is easily diffused in the lengthwise direction through the plurality of first grooves and the plurality of second grooves, the absorbent core can be more efficiently utilized in the present absorbent article.

### [Aspect 4]

The absorbent article according to any one of aspects 1 to 3, wherein
the inner layer has a plurality of inner layer grooves recessed in the thickness direction on the core facing surface.

Since the bodily fluid that has permeated between the absorbent core and the core facing surface of the inner layer is diffused through the plurality of inner layer grooves, the bodily fluid can be more efficiently diffused in the present absorbent article.

### [Aspect 5]

The absorbent article according to aspect 4, wherein
each of the plurality of inner layer grooves overlaps each of the plurality of first grooves.

Even in a case where the bodily fluid is repeatedly absorbed, the plurality of inner layer grooves are prevented from being deformed together with the plurality of first grooves. Therefore, in the present absorbent article, since a large amount of bodily fluid can be diffused through each of the plurality of first grooves overlapping each of the plurality of inner layer grooves, the liquid absorbency can be maintained at a higher level.

### [Aspect 6]

The absorbent article according to any one of aspects 1 to 5, wherein
the inner layer is formed of a tissue, and the outer layer is formed of a spunbond nonwoven fabric.

Since the inner layer is formed of a tissue, the bodily fluid can be more easily diffused into the inside of the inner layer. The outer layer is formed of a spunbond nonwoven fabric, which makes it possible to more reliably form the second diffusion space and the third diffusion space. Therefore, in the present absorbent article, the liquid absorbency can be maintained at a higher level. In a case where each of the inner layer grooves overlaps each of the plurality of first grooves, the low strength tissue is supported by the spunbond nonwoven fabric, which makes it possible to use the tissue having excellent hydrophilicity as the core-wrapping sheet. Since the extensibility of the tissue is small compared with that of the spunbond nonwoven fabric, in a case where processing is performed to the plurality of first grooves and the plurality of second grooves are formed in a state where the inner layer and the outer layer overlap each other, the tissue cannot follow deformation of the spunbond nonwoven fabric and tearing partially occurs in the tissue to form the plurality of open holes. Therefore, since the plurality of open holes are formed in the inner layer at the same time as the plurality of first grooves and the plurality of second grooves are formed in the outer layer, the core-wrapping sheet can be efficiently formed.

### [Aspect 7]

The absorbent article according to aspect 6, wherein
the inner layer and the absorbent core are joined with a hot-melt adhesive. Since the inner layer is formed of a tissue, the fiber density is high, and the contact area between the inner layer and the hot-melt adhesive becomes large correspondingly, which increases the adhesion with the absorbent core. Since the strength of the outer layer is less likely to decrease even in a case where the bodily fluid is repeatedly absorbed, deformation of the plurality of first grooves and the plurality of second grooves can be suppressed. Therefore, in the present absorbent article, shape collapse can be suppressed even in a case where the bodily fluid is repeatedly absorbed.

### [Aspect 8]

The absorbent article according to any one of aspects 1 to 7, wherein
the outer layer and the absorbent core are joined through the plurality of open holes.

Due to the direct joining between the outer layer and the absorbent core, even in a case where the inner layer that absorbs the bodily fluid is deformed, the positional deviation between the outer layer and the absorbent core can be prevented. Therefore, in the present absorbent article, shape collapse can be more reliably suppressed even in a case where the bodily fluid is repeatedly absorbed.

### [Aspect 9]

The absorbent article according to any one of aspects 1 to 8, wherein
the absorbent core has a low-basis-weight portion in which a basis weight of an absorbent material is smaller than a surrounding region, and
the inner layer is colored in a different color from a color of the absorbent core.

In the absorbent body that does not absorb the bodily fluid, the position of the low-basis-weight portion can be confirmed by the color of the inner layer that has been transmitted through the low-basis-weight portion. Therefore, in a dry state, the user can use the low-basis-weight portion as an index for position alignment. Since the absorbent body that has absorbed the bodily fluid is wetted by the absorbed bodily fluid, the color of the inner layer that has been transmitted through the low-basis-weight portion is less likely to be visually recognized. Therefore, in a wet state, the user can visually recognize the absorption state of the bodily fluid.

### [Aspect 10]

The absorbent article according to any one of aspects 1 to 9, wherein the core-wrapping sheet is arranged on a non-skin facing surface side.

The bodily fluid excreted on the top sheet surface permeates the top sheet, and a part of the bodily fluid is absorbed by the absorbent core. The bodily fluid that has not been absorbed by the absorbent core reaches the first diffusion space from the non-skin facing surface of the absorbent core and also reaches the second diffusion space through the plurality of open holes in the inner layer. Due to the diffusivity in the inner layer containing 50 mass% or more of cellulose fibers, the bodily fluid is diffused into the inside of the inner layer, the first diffusion space, and the second diffusion space, respectively. Further, the bodily fluid permeates the outer layer and reaches the second surface to diffuse the bodily fluid through the third diffusion space. Therefore, in the present absorbent article, the bodily fluid can be more efficiently diffused through the inside of the inner layer, the first diffusion space, the second diffusion space, and the third diffusion space.

### [Aspect 11]

The absorbent article according to aspect 10, wherein
the inner layer has a plurality of inner layer grooves recessed in the thickness direction on the core facing surface, and each of the plurality of open holes is formed in each of the plurality of inner layer grooves.

While diffusing through the inner layer groove, the bodily fluid permeates between the non-core facing surface and the first surface through the plurality of open holes and diffused between the non-core facing surface and the first surface. Therefore, in the present absorbent article, the bodily fluid can be more efficiently diffused.

### [Aspect 12]

The absorbent article according to any one of aspects 1 to 11, wherein
the absorbent core includes a skin side layer including the skin facing surface, and a non-skin side layer including the non-skin facing surface,
the skin side layer or the non-skin side layer includes
   a plurality of groove portions including a plurality of main groove portions that penetrate in the thickness direction, and
   a plurality of bases, and
each of the plurality of groove portions and each of the plurality of bases are alternately arranged.

Since the excreted bodily fluid is transported to a position apart from the excrement portion of the absorbent core through the plurality of groove portions and is absorbed in a larger area, the absorbency of the entire absorbent core can be enhanced. Since the liquid is transported from the excrement portion, a decrease in the absorption power of the absorbent core in the vicinity of the excrement portion can be suppressed.

### [Aspect 13]

An absorbent article comprising:
a liquid-permeable top sheet;
a liquid-impermeable back sheet; and
an absorbent body that is arranged between the top sheet and the back sheet, wherein
the absorbent body includes
   an absorbent core having a skin facing surface and a non-skin facing surface on a side opposite to the skin facing surface, and having a lengthwise direction, a width direction, and a thickness direction, and
   a core-wrapping sheet that is arranged at least one side of the skin facing surface and the non-skin facing surface,
the absorbent core includes a skin side layer including the skin facing surface, and a non-skin side layer including the non-skin facing surface, the skin side layer or the non-skin side layer includes a plurality of groove portions including a plurality of main groove portions that penetrate in the thickness direction, and a plurality of bases, and each of the plurality of groove portions and each of the plurality of bases are alternately arranged,
the core-wrapping sheet has an inner layer and an outer layer,
the inner layer contains 50 mass% or more of cellulose fibers, and has a core facing surface in contact with the absorbent core, and a non-core facing surface on a side opposite to the core facing surface,
the outer layer contains 50 mass% or more of thermoplastic synthetic fibers and has a first surface in contact with the non-core facing surface, a second surface on a side opposite to the first surface, a plurality of first grooves recessed in the thickness direction on the first surface, and a plurality of second grooves recessed in the thickness direction on the second surface, and
each of the plurality of first grooves and each of the plurality of second grooves are alternately arranged with predetermined spaces.

Similar to aspect 1, the present absorbent article has the first diffusion space, the second diffusion space, and the third diffusion space, and thus the same effect as that of aspect 1 can be obtained.

### [Aspect 14]

A method of manufacturing a core-wrapping sheet for an absorbent body including an absorbent core and an absorbent core-wrapping sheet,
the core-wrapping sheet including
an outer layer containing 50 mass% or more of thermoplastic synthetic fibers, and having a first surface and a second surface on a side opposite to the first surface, and
an inner layer containing 50 mass% or more of cellulose fibers, and having a core facing surface and a non-core facing surface on a side opposite to the core facing surface,
the method comprising:
a step of preheating an outer layer continuous body formed of the outer layer; and
a step of transporting the preheated outer layer continuous body and an inner layer continuous body formed of the inner layer between a pair of shaping members including a first shaping member and a second shaping member in a state where the first surface and the non-core facing surface are in contact with each other, and forming a plurality of core facing grooves recessed in a thickness direction on the core facing surface, a plurality of non-core facing grooves recessed in the thickness direction on the second surface, and a plurality of open holes penetrating the inner layer continuous body from the core facing surface to the non-core facing surface in the inner layer continuous body.

The plurality of core facing grooves, the plurality of non-core facing grooves, and the plurality of open holes are formed in a state where the outer layer continuous body and the inner layer continuous body overlap each other, which makes it possible to easily manufacture the core-wrapping sheet in which the inner layer and the outer layer are integrated. In a case where at least one of the skin facing surface and the non-skin facing surface of the absorbent core is covered with the obtained core-wrapping sheet, the bodily fluid is diffused into the first diffusion space which is a space between the absorbent core and the inner layer, the second diffusion space which is a space between the inner layer and the outer layer, the third diffusion space having the second grooves, and the inside of the inner layer, and thus the bodily fluid can be more efficiently diffused.

Hereinafter, an absorbent article according to an embodiment will be described. In the present specification, unless otherwise specified, "viewing an object (for example, an absorbent article, an absorbent body, or an absorbent core) placed on a horizontal plane in an unfolded state from above or below the object in the thickness direction" will be referred to as a "plan view". In a case where the object is an absorbent article, viewing the absorbent article in the thickness direction from a top-surface sheet side in an unfolded state may be simply referred to as a "plan view". In a case where the object is an absorbent core, viewing the absorbent article in the thickness direction from a non-skin facing surface side of the absorbent core in an unfolded state may be simply referred to as a "plan view".

Various directions and the like used in the present specification are as follows, unless otherwise specified. The "longitudinal direction" refers to "a direction in which the length of a longitudinally long object in a plan view is long", the "width direction" refers to "a direction in which the length of the longitudinally long object in a plan view is short", and the "thickness direction" refers to "a direction vertical to the object placed on a horizontal plane in an unfolded state". The longitudinal direction, the width direction, and the thickness direction are in a relationship that is orthogonal to each other. It should be noted that, although each direction includes two opposite directions, when shown in the drawings, there is a case where only one direction is shown with respect to a direction orthogonal to the paper surface.

### (First embodiment)

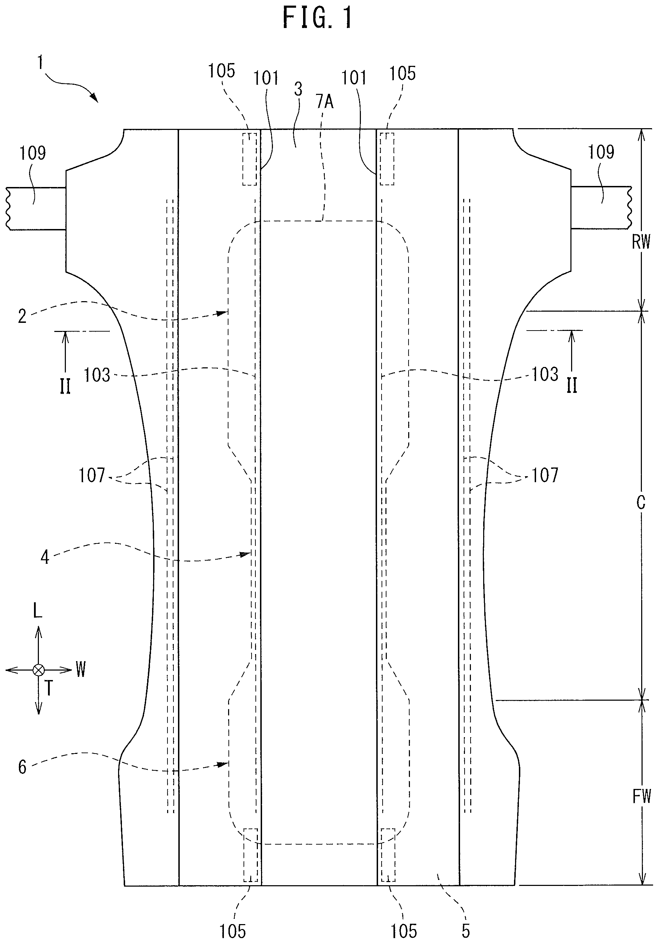

Hereinafter, an absorbent article according to a first embodiment will be described with reference to drawings. As shown in FIG. 1, an absorbent article 1 is an example of a disposable diaper and includes a liquid-permeable top sheet 3, a liquid-impermeable back sheet 5, and an absorbent body 7A that is arranged between the top sheet 3 and the back sheet 5. In a lengthwise direction L, the absorbent article 1 is divided into three regions: a front waist region FW, a rear waist region RW, and a crotch region C between the front waist region FW and the rear waist region RW. The absorbent article 1 further includes a pair of leakproof walls 101 including an elastic member 103, a fixed portion 105 that fixes the leakproof wall 101 to the top sheet 3, an elastic member 107 arranged around the leg portion, a tape fastener 109, and the like. It should be noted that these are well-known in the art and will not be described here.

The absorbent body 7A absorbs and holds the bodily fluid excreted in the top sheet 3. The absorbent body 7A has a lengthwise direction L, a width direction W, and a thickness direction T. The absorbent body 7A is arranged across the three regions. The absorbent body 7A has a first end portion 2 and a second end portion 6 having the same length in the width direction W at both end portions in the lengthwise direction L, and a constricted portion 4 having a shorter length in the width direction W than the first end portion 2 and the second end portion 6 at the central part in the lengthwise direction L. The first end portion 2 and the constricted portion 4, and the second end portion 6 and the constricted portion 4 are respectively connected to each other through inclined sides inclined toward the inner side in the width direction W toward the constricted portion 4.

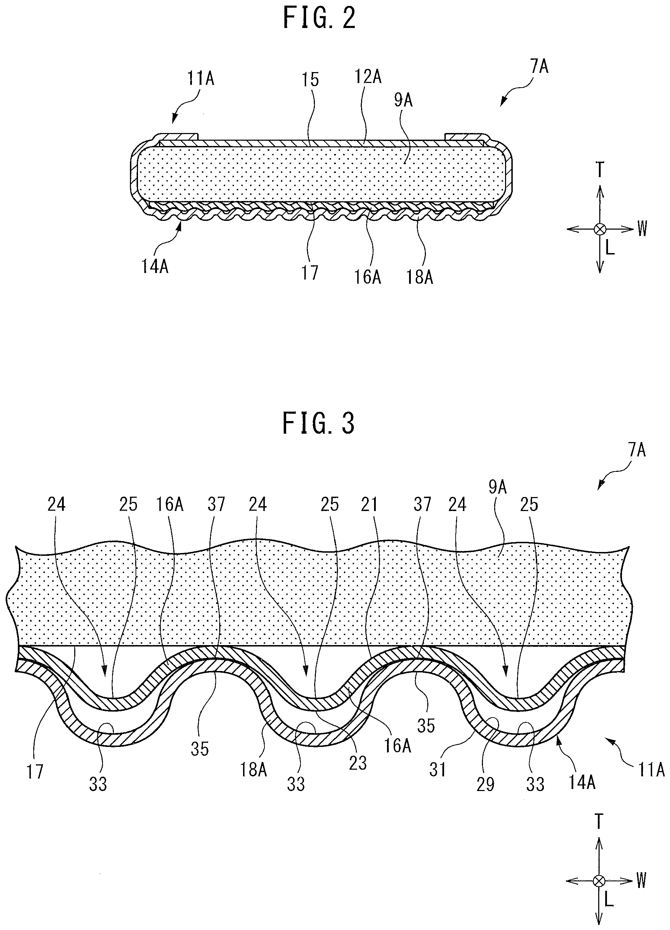

As shown in FIG. 2, the absorbent body 7A includes an absorbent core 9A and a core-wrapping sheet 11A. It should be noted that, in the present embodiment, since the lengthwise direction, the width direction, and the thickness direction of the absorbent article 1 are the same as the directions of the absorbent body 7A, the lengthwise direction L, the width direction W, and the thickness direction T are also used as the directions of the absorbent article 1. The absorbent core 9A includes water-absorbent fibers and superabsorbent polymer particles (SAP). As the water-absorbent fibers, pulp fibers can be used. The pulp fibers are not particularly limited as long as the fibers can be used for a sanitary product, and examples thereof include cellulose fibers. Examples of the cellulose fibers include wood pulp (for example, coniferous pulp and hardwood pulp), crosslinked pulp, and non-wood pulp. The superabsorbent polymer particles are not particularly limited as long as the superabsorbent polymer particles can be used for a sanitary product, and examples thereof include polyacrylate-based, polysulfonate-based, and maleic anhydride-based polymer particles. The average basis weight of the water-absorbent fibers in the absorbent core 9A is preferably 50 to 450 g/m², and more preferably 80 to 350 g/m². The average basis weight of the superabsorbent polymer particles in the absorbent core 9A is preferably 100 to 500 g/m², and more preferably 150 to 400 g/m². The average basis weight of the absorbent core is measured according to the following measurement method. The sheet is cut into a size of 5 cm × 5 cm and is used as a sample. The mass is measured after a drying treatment in an atmosphere of 100°C or higher. Next, the measured mass is divided by the area of the sample to calculate the basis weight of the sample. The value obtained by averaging the basis weights of 10 samples is defined as the average basis weight of the absorbent core. A method of measuring the average basis weight of the superabsorbent polymer particles is as follows. Five samples having a predetermined length and width are cut out from the absorbent core (for example, 4 mm × 4 mm), the superabsorbent polymer particles contained in each sample are selected, the total mass of the superabsorbent polymer particles contained in each sample is measured, and the average basis weight is obtained by the average value obtained by dividing the measured value by the area of the sample. Further, the average density is obtained by dividing the average basis weight by the thickness of the sample described below.

The absorbent core 9A has a skin facing surface 15 and a non-skin facing surface 17 on the side opposite to the skin facing surface 15, and has the lengthwise direction L, the width direction W, and the thickness direction T. The absorbent core 9A has an outer shape substantially identical to the outer shape of the absorbent body 7A. The thickness of the absorbent core 9A is preferably 0.2 to 10 mm, more preferably 1.0 to 8.0 mm, and more preferably 2.0 to 6.0 mm. It should be noted that, unless otherwise specified in the present specification, the thickness (mm) of the object (for example, an absorbent body or an absorbent core) refers to the average of five measured values obtained by pressurizing five different portions of the absorbent body in a standard state (temperature: 23 ± 2°C, relative humidity: 50 ± 5%) with FS-60DS (measurement surface: 44 mm (diameter), measurement pressure: 3 g/cm²) manufactured by Daiei Kagaku Seiki MFG, Co., Ltd., and measuring the thickness after 10 seconds of pressurization at each portion.

The core-wrapping sheet 11A encloses the absorbent core 9A. For example, the core-wrapping sheet 11A may be glued to the absorbent core 9A with a plurality of linear or dot-shaped adhesive portions (not shown). The adhesive portion can be formed of, for example, a hot-melt adhesive. The core-wrapping sheet 11A has an upper core-wrapping sheet 12A and a lower core-wrapping sheet 14A. The upper core-wrapping sheet 12A covers the skin facing surface 15 of the absorbent core 9A. The upper core-wrapping sheet 12A has the same outer shape as that of the skin facing surface 15 or slightly smaller than that of the skin facing surface 15, and is formed of a hydrophilic nonwoven fabric similar to the outer layer described below. The lower core-wrapping sheet 14A covers the non-skin facing surface 17 and the side surfaces of the absorbent core 9A. The lower core-wrapping sheet 14A is joined to the upper core-wrapping sheet 12A on the skin facing surface 15 in a state where the end portion of the lower core-wrapping sheet 14A in the width direction W overlaps the end portion of the upper core-wrapping sheet 12A in the width direction W, while covering the side surfaces from the non-skin facing surface 17.

As shown in FIG. 3, the lower core-wrapping sheet 14A has an inner layer 16A and an outer layer 18A. The inner layer 16A is in contact with the absorbent core 9A. The inner layer 16A is joined to the absorbent core 9A with the hot-melt adhesive. The inner layer 16A includes a nonwoven fabric which is an aggregate layer of fibers containing 50 mass% or more of cellulose fibers. Examples of the cellulose fibers include a wood pulp (for example, coniferous pulp or hardwood pulp), a crosslinked pulp, and a non-wood pulp, but the cellulose fibers are not limited thereto. The inner layer 16A may contain thermoplastic synthetic fibers or a paper strengthening agent in addition to the cellulose fibers. The thickness of the inner layer 16A is preferably 0.1 to 0.5 mm. The inner layer 16A may be formed of a tissue. The tissue refers to a tissue paper having a basis weight of 10 g/m² to 20 g/m² to which wet strength is imparted. The inner layer 16A has a core facing surface 21 facing the absorbent core 9A and a non-core facing surface 23 on the side opposite to the core facing surface 21. The inner layer 16A has the same outer shape as that of the absorbent core 9A, has an outer shape slightly smaller than that of the absorbent core, or has the same outer shape as that of the outer layer 18A. The inner layer 16A may have a plurality of inner layer grooves 25 recessed in the thickness direction T on the core facing surface 21. Each of the plurality of inner layer grooves 25 extends in the lengthwise direction L and is located with predetermined spaces in the width direction W.

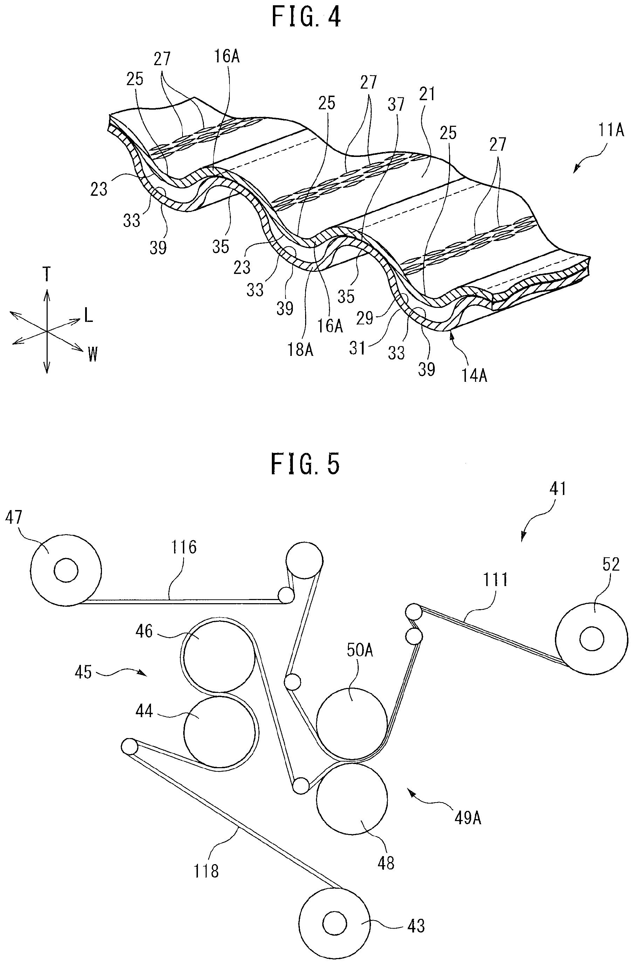

As shown in FIG. 4, the inner layer 16A has open holes 27 that penetrate the inner layer 16A in the thickness direction. The open hole 27 penetrates between the core facing surface 21 and the non-core facing surface 23 and communicates from the core facing surface 21 to the non-core facing surface 23. Unlike the pores formed between the fibers, the open holes 27 are intentionally formed in the inner layer 16A and have a much larger area than the pores formed between the fibers. A plurality of open holes 27 are respectively continuous in the lengthwise direction L, and a plurality of columns are arranged between the bottom portion and the apex portion of the inner layer groove 25. The plurality of open holes 27 may be mainly formed in the middle stomach between the bottom portion and the apex portion of the inner layer groove 25. The arrangement and shape of the open holes 27 shown in FIG. 4 are uniform, but in practice, it is not necessary that the arrangement and shape of the open holes 27 are uniform. The shape of the open hole 27 is generally a circular shape, an elliptical shape extending in the lengthwise direction L, or a rhombic shape. The open holes 27 do not have to be symmetrical. The open hole 27 may not be a completely opened space. The open holes 27 may be in a state where fibers that are stretched across the internal space and a part of the fibers that extend out are mixed with each other. The length of the open holes 27 in the lengthwise direction L is 0.1 to 5 mm, and preferably 0.2 to 2 mm, and the length of the open holes 27 in the width direction W is 0.05 to 3 mm, and preferably 0.1 to 1 mm. The core facing surface 21 of the inner layer 16A is in contact with the non-skin facing surface 17 at the apex portion between the inner layer groove 25 and the inner layer groove 25. The porosity of the internal space of the open hole 27 is preferably 1% to 50%, more preferably 1.5% to 35%, and still more preferably 2.5% to 20%.

The outer layer 18A is arranged on the side opposite to the absorbent core 9A of the inner layer 16A (FIG. 3). The outer layer 18A has an outer shape that covers the non-skin facing surface 17 and the side surfaces of the absorbent core 9A. The outer layer 18A may not be joined to the inner layer 16A. The outer layer 18A is formed of a hydrophilic nonwoven fabric. The outer layer 18A may contain, for example, any one or more of a spunbond nonwoven fabric, a meltblown nonwoven fabric, an air-through nonwoven fabric, and an air-laid nonwoven fabric. The outer layer 18A contains 50 mass% or more of thermoplastic synthetic fibers. Examples of the thermoplastic synthetic fibers include synthetic fibers of polyolefins such as polyethylene, polypropylene, polybutylene, ethylene-vinyl acetate copolymer, ethylene-ethyl acrylate copolymer, ethylene-acrylic acid copolymer, and ionomer resin; polyesters such as polyethylene terephthalate, polybutylene terephthalate, polytrimethylene terephthalate, and polylactic acid; and polyamides such as nylon. Examples of other constituent fibers include wood or non-wood pulp fibers, natural fibers (for example, wool, cotton, and the like), regenerated cellulose fibers (for example, rayon, acetate, and the like), and inorganic fibers (for example, glass fibers, carbon fibers, and the like). The fibers that constitute the outer layer 18A may have a form such as: composite fibers such as core-sheath fibers, side-by-side fibers, or island/sea fibers; hollow type fibers; irregularly shaped fibers such as flat fibers, Y-shaped fibers or C-shaped fibers; three-dimensional crimped fibers such as latent crimped or developed crimped fibers; or split fibers that are split by a physical load such as a water jet, heat and embossing. The basis weight of the outer layer is usually 10 to 75 g/m², preferably 10 to 45 g/m², and more preferably 10 to 25 g/m². The thickness of the outer layer is usually 0.1 to 5 mm, preferably 0.2 to 3 mm, and more preferably 0.4 to 2 mm. As the outer layer 18A, an SMS nonwoven fabric having a three-layer structure in which a meltblown nonwoven fabric is sandwiched between spunbond nonwoven fabrics, or an SMMS nonwoven fabric having a four-layer structure in which two meltblown nonwoven fabrics are sandwiched between spunbond nonwoven fabrics may be used.

As shown in FIG. 4, the outer layer 18A has a first surface 29 that faces the non-core facing surface 23, a second surface 31 that is arranged on a side opposite to the first surface 29, a plurality of first grooves 33 that are recessed in the thickness direction T on the first surface 29; and a plurality of second grooves 35 (also referred to as non-core facing grooves) that are recessed in the thickness direction T on the second surface 31. The plurality of first grooves 33 and the plurality of second grooves 35 are each alternately arranged with predetermined spaces. Each of the plurality of first grooves 33 and each of the plurality of second grooves 35 extend in a direction parallel to the lengthwise direction L and are alternately arranged with predetermined spaces in a direction parallel to the width direction W. The second groove 35 forms a first ridge 37 that protrudes in the thickness direction T when viewed from the first surface 29. The first ridge 37 is arranged between the first grooves 33 and is in contact with the non-core facing surface 23. The first ridge 37 may be joined to the non-skin facing surface 17 with the above-described adhesive portion through the open holes 27. The first groove 33 forms a second ridge 39 that protrudes in the thickness direction T when viewed from the second surface 31. The second ridge 39 is arranged between the second grooves 35 and is in contact with the back sheet 5 (not shown in the drawing). Each of the plurality of first grooves 33 may overlap each of the plurality of inner layer grooves 25. The first groove 33 and the inner layer groove 25 in an overlapped state will be referred to as a core facing groove 24 (FIG. 3). The non-core facing surface 23 and the first surface 29 are not joined to each other.

The height from the bottom portion of the first groove 33 to the apex portion of the first ridge 37 (hereinafter, referred to as "upper surface side ridge portion height") of the outer layer 18A is preferably 0.1 to 5 mm, more preferably 0.2 to 3 mm, and still more preferably 0.25 to 2 mm. It should be noted that the apex portion of the first ridge 37 is positioned at the substantially central part of the first ridge 37 in the width direction W. On the first surface 29 side, the space between the apex portions of the first ridges 37 which are adjacent to each other (hereinafter, referred to as an "upper surface side ridge portion space") is preferably 0.25 to 5 mm, more preferably 0.5 to 3 mm, and still more preferably 0.75 to 2 mm. In the upper surface side ridge portion height and the upper surface side ridge portion space in these ranges, spaces that function as liquid transport channels are formed between the inner layer 16A and the outer layer 18A and between the outer layer 18A and the back sheet 5, and the bodily fluid can be drawn and diffused into the spaces. It is preferable that, from the viewpoint of enhancing liquid diffusivity, the plurality of first ridges 37 and the first grooves 33 extend in a manner of continuing from one end edge to the other end edge of the outer layer 18A in a direction parallel to the lengthwise direction L of the absorbent core 9A. The upper surface side ridge portion height and the upper surface side ridge portion space are measured using a two-dimensional laser displacement meter. As the two-dimensional laser displacement meter, for example, a high precision two-dimensional laser displacement meter LJ-G series (model: LJ-G030) manufactured by Keyence Corporation may be used.

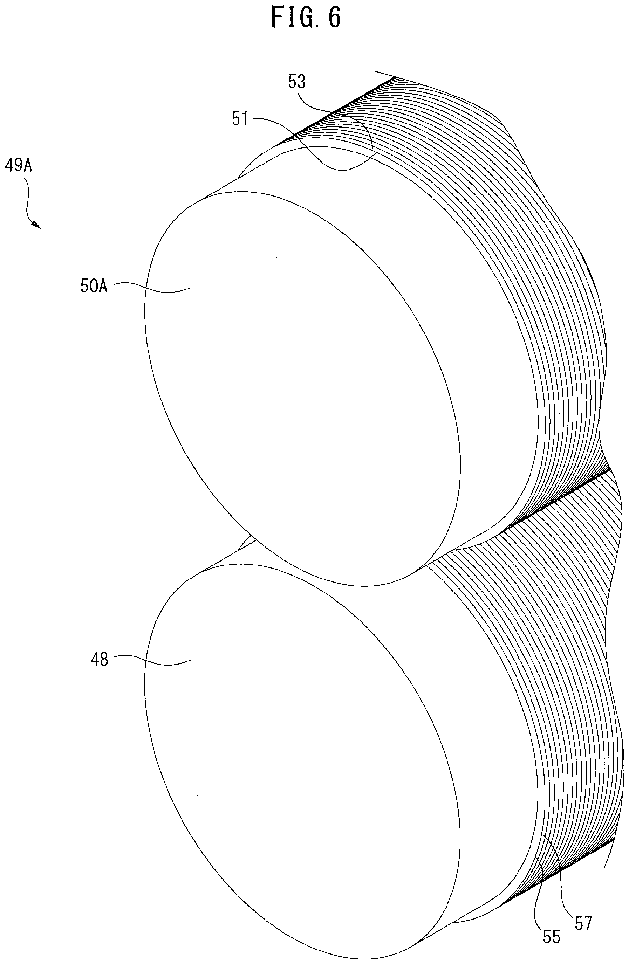

Hereinafter, a method of manufacturing the core-wrapping sheet 11A will be described. FIG. 5 is a schematic view showing an example of a manufacturing apparatus for manufacturing the core-wrapping sheet according to the first embodiment. FIG. 6 is a schematic view showing a shaping roll that can be used in the manufacturing apparatus for manufacturing the core-wrapping sheet according to the first embodiment. A manufacturing apparatus 41 shown in FIG. 5 includes a first unwinding device 43, a preheating device 45, a second unwinding device 47, a shaping device 49A, and a winding device 52. In the first unwinding device 43, an outer layer continuous body 118 formed of the outer layer 18A is wound in a roll form, and the outer layer continuous body 118 is unwound in the transport direction. The preheating device 45 preheats the unwound outer layer continuous body 118. In the present embodiment, the preheating device 45 includes a pair of upper and lower heating rolls 44 and 46. The preheating device 45 winds and heats the transported outer layer continuous body 118 around the rotating lower heating roll 44. After the outer layer continuous body 118 is heated by the lower heating roll 44, the outer layer continuous body 118 is transferred to the rotating upper heating roll 46, and the outer layer continuous body 118 is heated again by the upper heating roll 46. In the second unwinding device 47, the inner layer continuous body 116 formed of the inner layer 16A is wound in a roll form, and the inner layer continuous body 116 is unwound in the transport direction.

As shown in FIG. 6, the shaping device 49A includes a pair of upper and lower stretching rolls 48 and 50A. The upper stretching roll 50A includes a plurality of protruding ridges 51 and a plurality of recessed grooves 53 respectively arranged between the plurality of protruding ridges 51. The plurality of protruding ridges 51 and the plurality of recessed grooves 53 extend in a circumferential direction and are alternately arranged with regular spaces in a roll width direction. On the other hand, the lower stretching roll 48 includes, on the outer circumferential surface, a plurality of recessed grooves 57 located so as to engage the protruding ridges 51 of the upper stretching roll 50A, and a plurality of protruding ridges 55 located so as to engage the recessed grooves 53 of the upper stretching roll 50A.

In a case of manufacturing the core-wrapping sheet 11A using the manufacturing apparatus 41, a preheating step of preheating the outer layer continuous body 118 unwound from the first unwinding device 43, and a shaping step of shaping the outer layer continuous body 118 that has undergone the preheating step and an inner layer continuous body 116 that has been unwound from the second unwinding device 47 in an overlapped state are sequentially performed. In the preheating step, the outer layer continuous body 118 is preheated by sequentially contacting the outer circumferential surfaces of the pair of upper and lower heating rolls 44 and 46. The preheating temperature is a temperature lower than the melting point of the thermoplastic synthetic fibers contained in the outer layer continuous body 118. For example, in a case where the thermoplastic synthetic fibers contained in the outer layer continuous body 118 are sheath-core type composite fibers of polyethylene terephthalate (PET) and high density polyethylene (HDPE), it is preferable that the preheating temperature of the outer circumferential surface of the heating roll is approximately 60°C to 120°C.

In the shaping step, the inner layer continuous body 116 and the outer layer continuous body 118 transported through the preheating step are transported between the pair of upper and lower stretching rolls 48 and 50A that are rotating while being engaged in the shaping device 49A in a state where the non-core facing surface 23 and the first surface 29 are in contact with each other. The pair of upper and lower stretching rolls 48 and 50A stretch and shape the inner layer continuous body 116 and the outer layer continuous body 118 between the protruding ridges 51 and the recessed grooves 53 of the upper stretching roll 50A and the recessed grooves 57 and the protruding ridges 55 of the lower stretching roll 48, which are engaged with each other. At this time, the upper stretching roll 50A forms the core facing grooves 24 by pushing a portion in which the protruding ridges 51 are in contact with the inner layer continuous body 116 into the recessed grooves 57 of the lower stretching roll 48. The lower stretching roll 48 forms the non-core facing grooves (second grooves) 35 by pushing a portion in which the protruding ridges 55 are in contact with the outer layer continuous body 118 into the recessed grooves 53 of the upper stretching roll 50A. It should be noted that, in the shaping step, shaping may be performed while the stretching rolls 48 and 50A are heated to 60°C to 120°C in order to facilitate shaping. A core-wrapping sheet continuous body 111 from which the shaping step has been completed is wound by the winding device 52, and by using the core-wrapping sheet continuous body 111 as the core-wrapping sheet 11A for the absorbent article 1, the absorbent article 1 can be manufactured according to a well-known method.

In the shaping step, the protruding ridges 51 of the upper stretching roll 50A are pushed into the recessed grooves 57 of the lower stretching roll 48, and at the same time, the protruding ridges 55 of the lower stretching roll 48 are pushed into the recessed grooves 53 of the upper stretching roll 50A. The first grooves 33 and the second grooves 35 are formed by deforming the outer layer continuous body 118 along the protruding ridges 51 and the protruding ridges 55 while the outer layer continuous body 118 is stretched in portions between the protruding ridges 51 and the protruding ridges 55 (hereinafter, referred to as an "engaged portions").

In a case where the inner layer continuous body 116 is formed of a tissue and the outer layer continuous body 118 is formed of a spunbond nonwoven fabric, the extensibility of the tissue is small compared with the spunbond nonwoven fabric. Therefore, the inner layer continuous body 116 is not stretched in the same manner as the outer layer continuous body 118, and therefore, the inner layer continuous body 116 is partially torn at the engaged portions. In this manner, the inner layer continuous body 116 cannot follow the deformation of the outer layer continuous body 118, the cellulose fibers are broken to cause partial tearing on the surface, and thereby a plurality of open holes 27 that penetrate the inner layer continuous body 116 are formed. Therefore, since the plurality of open holes 27 can be formed simultaneously with the plurality of core facing grooves 24, the core-wrapping sheet 11A can be more efficiently manufactured.

A description will be given of operation and effects of the above-described absorbent article 1. First, the bodily fluid excreted on the surface of the top sheet 3 permeates the top sheet 3. The bodily fluid that has permeated the top sheet 3 permeates from the skin facing surface 15 to the inside of the absorbent core 9A through the upper core-wrapping sheet 12A, and a part of the bodily fluid is absorbed by the absorbent core 9A. The bodily fluid not absorbed by the absorbent core 9A exudes from the non-skin facing surface 17, reaches the first diffusion space between the non-skin facing surface 17 and the core facing surface 21, and also reaches the second diffusion space between the non-core facing surface 23 and the first surface 29 through the plurality of open holes 27 in the inner layer 16A. Since the inner layer 16A contains 50 mass% or more of cellulose fibers, the diffusivity is excellent, and therefore, the bodily fluid is diffused into the inside of the inner layer 16A, the first diffusion space, and the second diffusion space, respectively. Further, while the bodily fluid that has reached the first surface 29 is diffused through the first grooves 33, the bodily fluid permeates the inside of the outer layer 18A and reaches the third diffusion space between the second surface 31 and the back sheet 5. The bodily fluid that has reached the space between the second surface 31 and the back sheet 5 is diffused through the third diffusion space. Therefore, in the absorbent article 1, in addition to the first diffusion space, the second diffusion space, and the third diffusion space, the bodily fluid can be more efficiently diffused due to the capillary phenomenon that occurs inside of the inner layer 16A, which makes it possible to maintain the liquid absorbency at a higher level. Since the outer layer 18A contains 50 mass% or more of thermoplastic synthetic fibers, the strength is less likely to decrease even in a case where the bodily fluid is repeatedly absorbed, deformation of the plurality of first grooves 33 and the plurality of second grooves 35 can be suppressed. Therefore, in the absorbent article 1, a large amount of bodily fluid can be diffused through the plurality of first grooves 33 and the plurality of second grooves 35 even in a case where the bodily fluid is repeatedly absorbed.

In a case where the non-core facing surface 23 and the first surface 29 are not joined to each other, the second diffusion space is more reliably secured. Therefore, the bodily fluid can be more reliably diffused into the second diffusion space. In a case where the inner layer groove 25 is provided, the bodily fluid that has permeated the core facing surface 21 of the absorbent core 9A and the inner layer 16A can be diffused in the inner layer groove 25. In a case where the inner layer groove 25 has the core facing groove 24 that overlaps the first groove 33, the inner layer groove 25 of the core facing groove 24 is held by the first groove 33, and thus deformation of the core facing groove 24 can be suppressed even in a case where the bodily fluid is repeatedly absorbed. Therefore, in the absorbent article 1, a large amount of bodily fluid can be diffused through the core facing groove 24. In a case where the inner layer 16A is formed of a tissue and the inner layer 16A and the absorbent core 9A are joined with the hot-melt adhesive, the tissue of the inner layer 16A has a high fiber density, the contact area between the inner layer 16A and the hot-melt adhesive becomes large correspondingly, and therefore the adhesion with the absorbent core 9A is high. Therefore, in the absorbent article 1, shape collapse can be suppressed even in a case where the bodily fluid is repeatedly absorbed. The outer layer 18A having the plurality of first ridges 37 (second grooves 35) and the plurality of first grooves 33 as described above enhances the flexibility and stretchability of the outer layer 18A, which makes it possible to enhance the ability of the core-wrapping sheet 11A to be deformed in accordance with the movement of the wearer and to enhance the feeling of wearing of the absorbent article 1. In a case where the first ridges 37 are joined to the non-skin facing surface 17 by the adhesive portions through the open holes 27, the positional deviation between the outer layer 18A and the absorbent core 9A can be prevented even when the inner layer 16A that has absorbed the bodily fluid is deformed.

The absorbent article of the present invention is not limited to the above-described embodiments and these can be appropriately combined, modified, or the like without departing from the scope of the present invention. For example, the case in which the inner layer grooves 25, the first grooves 33, and the second grooves 35 extend in the lengthwise direction L has been described. However, the inner layer grooves 25, the first grooves 33, and the second grooves 35 may be formed so as to extend in the width direction W. In this case, in the shaping step shown in FIG. 5, a pair of stretching rolls (not shown) in which the directions of the plurality of protruding ridges and the plurality of recessed grooves are different from each other may be used instead of the pair of stretching rolls 48 and 50A shown in FIG. 6. For example, the plurality of protruding ridges and the plurality of recessed grooves of the upper stretching roll are stretched in the width direction of the roll and are alternately arranged with regular spaces in the circumferential direction of the roll. On the other hand, the lower stretching roll includes, on the outer circumferential surface, a plurality of recessed grooves located so as to engage the protruding ridges of the upper stretching roll, and a plurality of protruding ridges located so as to engage the recessed grooves of the upper stretching roll. In a case where the inner layer grooves 25, the first grooves 33, and the second grooves 35 extend in the width direction W, the open holes may have an elliptical or rhombic shape that is elongated in the width direction W.

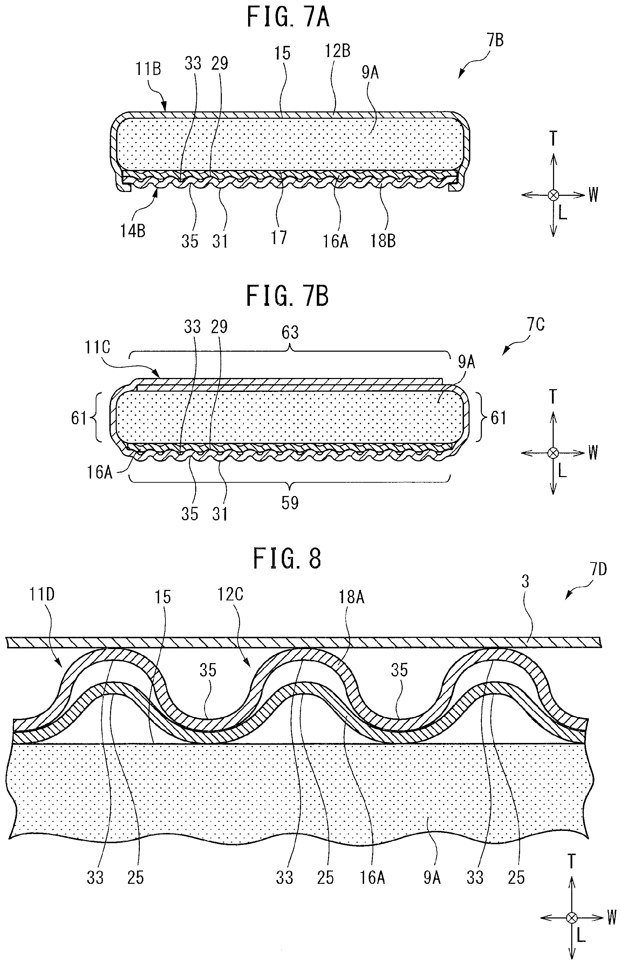

FIG. 7A is a cross-sectional view of an absorbent body 7B according to modified example (1) of the first embodiment taken along a line II-II in FIG. 1. A core-wrapping sheet 11B may be joined to the non-skin facing surface 17. The core-wrapping sheet 11B includes an upper core-wrapping sheet 12B and a lower core-wrapping sheet 14B. The lower core-wrapping sheet 14B has the same outer shape as that of the non-skin facing surface 17 or slightly smaller than that of the non-skin facing surface 17, and has the inner layer 16A and the outer layer 18B. The upper core-wrapping sheet 12B covers the skin facing surface 15 and the side surfaces. The upper core-wrapping sheet 12B is joined to the lower core-wrapping sheet 14B on the non-skin facing surface 17 in a state where the end portion in the width direction W overlaps the end portion of the lower core-wrapping sheet 14B in the width direction W while covering the side surface from the skin facing surface 15.

FIG. 7B is a cross-sectional view of an absorbent body 7C according to modified example (2) of the first embodiment taken along a line II-II in FIG. 1. As a core-wrapping sheet 11C, an integrated sheet in which the upper core-wrapping sheet and the lower core-wrapping sheet are integrated may be used. The core-wrapping sheet 11C formed of the integrated sheet includes a non-skin facing region 59 that covers the non-skin facing surface 17, a pair of side surface regions 61 that cover the side surfaces, and a pair of skin facing regions 63 that cover the skin facing surface 15. The non-skin facing region 59 is arranged at the center of the core-wrapping sheet 11C in the width direction W. The inner layer 16A is arranged between the core-wrapping sheet 11C of the non-skin facing region 59 and the absorbent core 9A. In the non-skin facing region 59, the core-wrapping sheet 11C has the first surface 29 that is in contact with the non-core facing surface 23, the second surface 31 that is on the side opposite to the first surface 29, the plurality of first grooves 33 that are recessed in the thickness direction T on the first surface 29, and the plurality of second grooves 35 that are recessed in the thickness direction T on the second surface 31. Each of the pair of side surface regions 61 is arranged in a manner of continuing at both width end portions of the non-skin facing region 59 in the width direction W. Each of the pair of skin facing regions 63 is arranged in a manner of continuing at an end portion of each of the pair of side surface regions 61 in the width direction W. In the core-wrapping sheet 11C, the non-skin facing surface 17 is covered with the non-skin facing region 59 together with the inner layer 16A, both side surfaces of the absorbent core 9A are covered with the pair of side surface regions 61, and in a state where the pair of skin facing regions 63 overlaps the skin facing surface 15, the pair of skin facing regions 63 are joined to each other in the skin facing surface 15.

The core-wrapping sheet is not limited to a case of covering the entire absorbent core 9A including the non-skin facing surface 17, and may cover only the non-skin facing surface 17 or may cover only the skin facing surface 15. The core-wrapping sheet may cover the skin facing surface 15 and the non-skin facing surface 17 excluding the side surfaces of the absorbent core 9A.

FIG. 8 is a partially enlarged cross-sectional view of an absorbent body 7D according to modified example (3) of the first embodiment. A core-wrapping sheet 11D includes an upper core-wrapping sheet 12C that covers the skin facing surface 15. The upper core-wrapping sheet 12C has the inner layer 16A facing the absorbent core 9A and the outer layer 18A arranged on the side opposite to the absorbent core 9A of the inner layer 16A. The outer layer 18A is in contact with the top sheet 3. In this case, the bodily fluid that has excreted on the surface of the top sheet 3 and permeated the top sheet 3 reaches the third diffusion space between the top sheet 3 and the outer layer 18A, permeates the outer layer 18A, and also reaches the second diffusion space between the outer layer 18A and the inner layer 16A. Further, the bodily fluid reaches the first diffusion space between the inner layer 16A and the absorbent core 9A through the plurality of open holes 27 of the inner layer 16A (not shown in the drawing). Due to the diffusivity in the inner layer 16A, the bodily fluid is diffused into the inside of the inner layer 16A, the first diffusion space, and the second diffusion space, respectively. Further, the bodily fluid is diffused through the third diffusion space having the second grooves 35. Therefore, the same effect as that of the first embodiment can be obtained.

The open hole in the inner layer may be formed, for example, by piercing a needle in the thickness direction of the inner layer. In this manner, the inner layer having the open holes formed in advance as described above may overlap the outer layer having the first grooves and the second grooves and may be used as the core-wrapping sheet.

In the first embodiment, the case where the absorbent article is a disposable diaper has been described, but the present invention is not limited thereto. Examples of the absorbent article include absorbent articles that mainly absorb urine, for example, urine absorption pads, animal urination sheets, and the like, and absorbent articles that mainly absorb menstrual blood and the like, for example, sanitary napkins, panty liners, and the like.

### 2. Second Embodiment

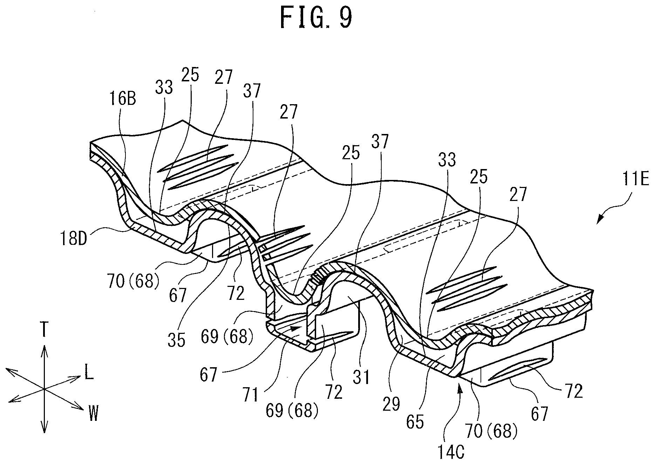

An absorbent article according to a second embodiment has a different core-wrapping sheet from the first embodiment. The same reference signs are given to the same configurations as in the first embodiment, and the description thereof is omitted. It should be noted that the core-wrapping sheet shown in FIG. 9 shows only the lower core-wrapping sheet, but the core-wrapping sheet may be applied to the upper core-wrapping sheet as shown in FIG. 8.

A core-wrapping sheet 11E shown in FIG. 9 includes a lower core-wrapping sheet 14C including an inner layer 16B and an outer layer 18D. An outer layer 18D has the first surface 29 and the second surface 31, and has the plurality of first grooves 33 on the first surface 29 and the plurality of second grooves 35 on the second surface 31. Between the first grooves 33, the first ridges 37 that protrude in the thickness direction T from the first surface 29 are located. The first grooves 33 and the first ridges 37 extend in a direction parallel to the lengthwise direction L and are alternately located with predetermined spaces in the width direction W. The first groove 33 has a first bottom portion 65 at the center in the width direction W. A recessed portion 67 is formed in the first bottom portion 65. The recessed portions 67 are recessed in the thickness direction T from the first bottom portion 65 and are located discontinuously in the lengthwise direction L. Each recessed portion 67 has a circumferential wall portion 68 and a second bottom portion 71. The circumferential wall portion 68 includes a pair of first circumferential wall portions 69 that extend in the lengthwise direction L and face each other, and a pair of second circumferential wall portions 70 that extend in the width direction W and face each other.

The circumferential wall portion 68 may have a hole portion 72 that penetrates the circumferential wall portion 68 and communicates with the second surface 31. Since the bodily fluid can be transferred from the first surface 29 side toward the second surface 31 side of the outer layer 18D through the hole portions 72, the transition of the bodily fluid from the first surface 29 side toward the second surface 31 side of the outer layer 18D can be promoted. The hole portion 72 is formed at a position close to the second bottom portion 71 of the recessed portion 67 in the first circumferential wall portion 69, and two hole portions 72 are present in each recessed portion 67. Such a hole portion 72 is not located in the second circumferential wall portion 70. The case where the hole portions 72 are located in the pair of first circumferential wall portions 69 has been described, but the hole portions 72 may be located in the pair of second circumferential wall portions 70 or may be located in any one of the circumferential wall portions 68.

It is preferable that, on the first surface 29 side, a distance from the height of the first bottom portion 65 to the height of the upper surface side of the second bottom portion 71 (hereinafter, referred to as "upper surface side recessed portion depth") is 10% to 80%, more preferably 15% to 70%, and still more preferably 20% to 60% of the upper surface side ridge portion height. In a case where the upper surface side recessed portion depth is within the above range, a space having a sufficient volume for storing the bodily fluid is secured in the recessed portions 67. The length of the recessed portion 67 in the lengthwise direction L is preferably 0.25 to 5 mm, more preferably 0.5 to 3 mm, and still more preferably 0.75 to 2 mm. The length of the recessed portion 67 in the width direction W depends on the upper surface side ridge portion space, and is preferably 0.25 to 5 mm, more preferably 0.5 to 3 mm, and still more preferably 0.75 to 2 mm. When the length of the recessed portion 67 in the lengthwise direction L and/or the width direction W is within the above range, the effect of primarily storing the bodily fluid that has permeated the absorbent core can be sufficiently exhibited in the recessed portion 67. The upper surface side recessed portion depth, the length of the recessed portion 67 in the lengthwise direction L, and the length of the recessed portion 67 in the width direction W are measured using a two-dimensional laser displacement meter. As the two-dimensional laser displacement meter, for example, a high precision two-dimensional laser displacement meter LJ-G series (model: LJ-G030) manufactured by Keyence Corporation may be used.

Since the hole portion 72 is opened toward the second groove 35 that is adjacent to the recessed portion 67, and the second groove 35 can function as a liquid transport channel, the transition of the bodily fluid from the first surface 29 to the second surface 31 side and the transition from the second surface 31 side to the first surface 29 side can be promoted. Due to that, in the absorbent article, the diffusion of the bodily fluid in the in-plane direction of the outer layer 18D can be promoted. It is preferable that the hole portion 72 is formed without melting the thermoplastic synthetic fibers of the outer layer 18D. In a case of melting and cutting the thermoplastic synthetic fibers, since the end portions of the fibers are melted and rounded, the fiber diameter becomes large, and due to that, the capillary phenomenon is less likely to occur in the peripheral edge portion of the hole portion 72. Therefore, it is preferable that the hole portion 72 is formed by breaking the thermoplastic synthetic fibers. It is preferable that in the internal space of the hole portion 72, fibers that are stretched across the internal space and a part of the extended fibers are in a mixed state, and the space is not a completely opened space. The porosity of the internal space of the hole portion 72 is preferably 1% to 50%, more preferably 1.5% to 35%, and still more preferably 2.5% to 20%. When the porosity of the internal space of the hole portion 72 is within the above range, the effect caused by the hole portion 72 is sufficient, and a decrease in the strength of the first circumferential wall portion 69 in which the hole portion 72 is located can be suppressed. However, the porosity of the internal space of the hole portion 72 may be out of the above range and or may be optionally set depending on the type, application, and the like of the absorbent article of the present invention.

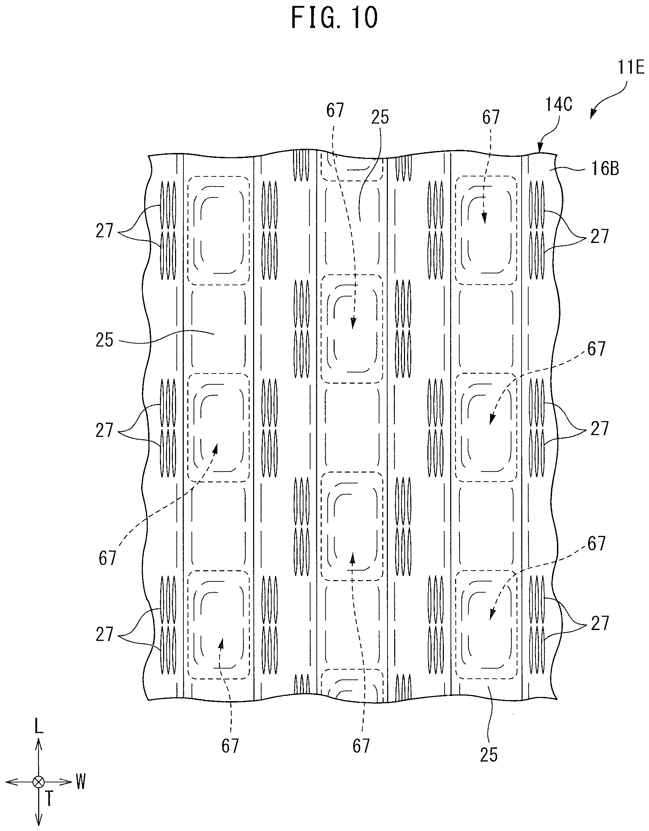

The inner layer groove 25 of the inner layer 16B overlaps the first groove 33. The inner layer 16B has a plurality of open holes 27 in the inner layer groove 25. The plurality of open holes 27 are arranged in a plurality of rows between the bottom portion and the apex portion of the inner layer groove 25 with predetermined spaces in the lengthwise direction L. As shown in FIG. 10, the open holes 27 are respectively arranged at the positions on both sides in the width direction W with respect to the recessed portion 67. As shown in FIG. 10, the open holes 27 may be arranged in two rows and three columns (six open holes) on both sides of the recessed portion 67 in the width direction W.

Since the core-wrapping sheet 11E is provided with the outer layer 18D that is recessed in two stages in a plan view, the bulk recovery property in the thickness direction is high. Therefore, the absorbent article provided with the core-wrapping sheet 11E can further maintain a high liquid absorption rate even in a case where the absorbent article is repeatedly exposed to a large amount of bodily fluid such as urine. Further, since the recessed portions 67 of the outer layer 18D have openings that are opened toward the absorbent core side, the bodily fluid that has permeated the absorbent core can be temporarily stored in the spaces within the recessed portions 67 while diffusing the bodily fluid through the first grooves 33 and the second grooves 35. The bodily fluid stored in the recessed portions 67 is diffused through the first grooves 33 and/or transferred to the second surface 31 side due to the capillary phenomenon, flows toward the second surface 31 side, and is diffused in the in-plane direction of the outer layer 18D. The bodily fluid that has transferred to the second surface 31 side diffuses and then again permeates the outer layer 18D and transfers to the first surface 29 side, transfers between the inner layer 16B and the absorbent core through the open holes 27, and is absorbed by the absorbent core.

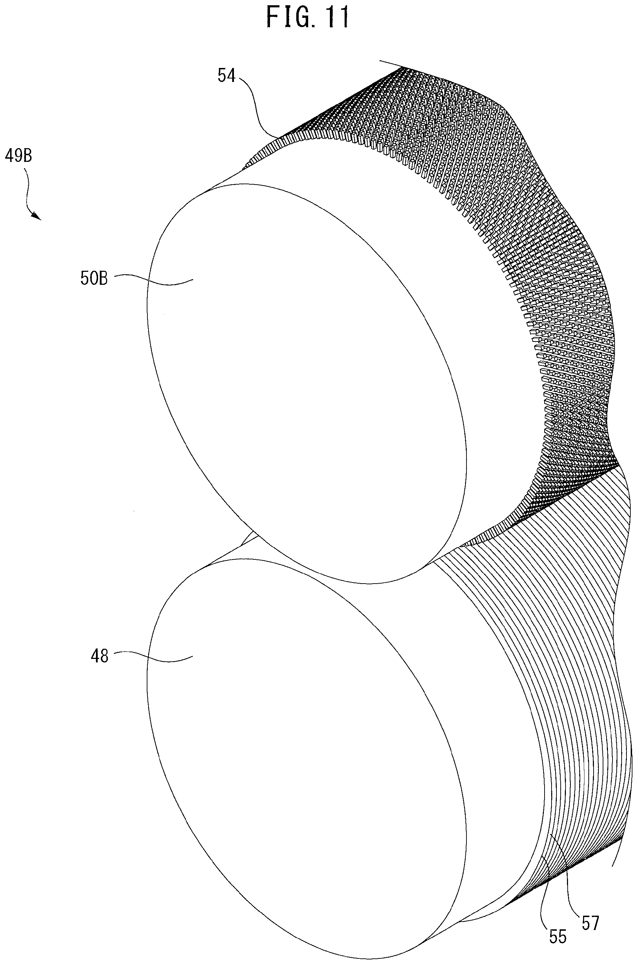

The core-wrapping sheet according to the present embodiment can be manufactured by replacing the shaping device 49A with a shaping device 49B shown in FIG. 11 in the manufacturing apparatus 41 shown in FIG. 5. The shaping device 49B includes a pair of upper and lower stretching rolls 48 and 50B. The lower stretching roll 48 includes a plurality of protruding ridges 55 and a plurality of recessed grooves 57 respectively arranged between the plurality of protruding ridges 55. The plurality of protruding ridges 55 and the plurality of recessed grooves 57 extend in the circumferential direction and are alternately arranged with regular spaces in the roll width direction. On the other hand, the upper stretching roll 50B includes a plurality of pins 54 located on the outer circumferential surface so as to be engaged with the recessed grooves 57 of the lower stretching roll 48. The pins 54 are arranged with regular spaces in the roll width direction so as not to come into contact with the protruding ridges 55 of the upper stretching roll 50B, and the pins 54 are arranged substantially linearly with regular spaces along the outer circumferential surface in the circumferential direction of the roll. In the upper stretching roll 50B, a plurality of pins 54 may be arranged in a zigzag shape on the outer circumferential surface of the upper stretching roll 50B.

In the shaping step, the lower stretching roll 48 pushes a portion in which the protruding ridges 55 are in contact with the outer layer continuous body 118 in the direction of the upper stretching roll 50B, and accordingly, the first ridges 37 (second grooves 35) are shaped. On the other hand, in the upper stretching roll 50B, the plurality of pins 54 arranged side by side in the circumferential direction push the inner layer continuous body 116, which is in contact with the leading end portions of the pins 54, into the identical recessed grooves 57 of the lower stretching roll 48. At this time, the portions of the inner layer continuous body 116 and the outer layer continuous body 118 that are pulled into the recessed grooves 57 in a non-contact state with the pins 54 function as the core facing grooves 24. Further, since the portions that have been in contact with the leading end portions of the pins 54 are strongly pushed and shaped into the recessed grooves 57, the recessed portions 67 having the first circumferential wall portions 69 in which the first ridges 37 and the first grooves 33 extend in the lengthwise direction L, and the second circumferential wall portions 70 and the second bottom portions 71 extending in the roll width direction are formed. It should be noted that the second bottom portion 71 of the recessed portion 67 is formed by pushing the contact portion of the outer layer continuous body 118 into the recessed groove 57 with the leading end portion of the pin 54 in a state where the upper stretching roll 50B and the lower stretching roll 48 bite the inner layer continuous body 116 and the outer layer continuous body 118 at the time of formation.

In the portions that have been in contact with both end portions of the leading end portion of the pin 54 in the width direction (roll width direction) in the outer layer continuous body 118, with the help of the tension generated when the protruding ridge 55 pushes the outer layer continuous body 118 in the direction of the upper stretching roll 50B, the pin 54 scrapes the thermoplastic synthetic fibers forming the first circumferential wall portion 69 or breaks the fibers to form broken fibers having broken end portions. Accordingly, in the recessed portion 67, the hole portion 72 including the broken end portions of the broken fibers is formed. It should be noted that a part of the thermoplastic synthetic fibers remain in a state of being stretched across the internal space of the hole portion 72, and the broken end portions of a part of the broken fibers extend into the internal space. Here, since the hole portion 72 is formed in the direction along the transport direction MD of the outer layer continuous body 118, that is, in the rotational direction of the stretching roll 48 and the direction in which the first ridge 37 and the first groove 33 extend, the hole portion 72 is also formed in the first circumferential wall portion 69 along the direction in which the first ridge 37 and the first groove 33 extend. The case where the recessed portion 67 is formed in a substantially rectangular parallelepiped shape has been described, but the shape of the recessed portion 67 can be any shape such as a cylindrical shape or a prism shape.

In a case where the inner layer continuous body 116 is formed of a tissue and the outer layer continuous body 118 is formed of a spunbond nonwoven fabric, the extensibility of the tissue is small compared with the spunbond nonwoven fabric. Therefore, since the inner layer continuous body is not stretched in the same manner as the outer layer continuous body 118, the inner layer continuous body is torn at the position where the leading end portion of the pin 54 comes into contact with the inner layer continuous body. In this manner, in particular, in the portion of the outer layer continuous body 118 in which the recessed portion 67 is formed, the inner layer continuous body 116 cannot follow the deformation of the outer layer continuous body 118, the cellulose fibers are broken to cause partial tearing, and thereby the plurality of open holes 27 are formed in the inner layer continuous body 116. It should be noted that a part of the cellulose fibers remain in a state of being stretched across the internal space of the open hole 27, and the broken end portions of the part of the broken cellulose fibers extend into the internal space. In FIGS. 9 and 10, a case where the open holes 27 are discontinuous in the lengthwise direction L, that is, partially formed at positions corresponding to the recessed portions 67 is shown. However, the configuration is not limited thereto, and the open holes 27 may also be formed between the recessed portions 67.

### 3. Third Embodiment

An absorbent article according to a third embodiment has an absorbent core different from that of the first embodiment. An absorbent core according to the third embodiment has a groove portion. The groove portion may extend in the lengthwise direction L or the width direction W, and may extend linearly, non-linearly, for example, in a curved shape. The groove portion may be formed in an annular shape in a plan view. The groove portion may include a main groove portion. The groove portion may include a main groove portion and a sub-groove portion. In the following description, the "main groove portion" means not only a groove portion that extends in a direction parallel to the lengthwise direction L, but also a groove portion that extends in the lengthwise direction L. An angle formed between the main groove portion and the lengthwise direction L is preferably less than 45°, more preferably less than 30°, and even more preferably less than 15°. The main groove portion can extend linearly, or non-linearly, for example, in a curved shape. In the present specification, the width of the main groove portion means the length of the main groove portion in a direction orthogonal to a direction in which the main groove portion extends in an object portion. The "sub-groove portion" means not only a groove portion that extends in a direction parallel or substantially parallel to the width direction W, but also a groove portion that extends in a direction other than the lengthwise direction L. In a case where the sub-groove portion communicates with the main groove portion, an angle formed between the sub-groove portion and the main groove portion (or the tangent line of the main groove portion) at the communicating portion (base end) is preferably 45° or more, more preferably 60° or more, and even more preferably 80° or more. The sub-groove portion can extend linearly, or non-linearly, for example, in a curved shape. In the present specification, the width of the sub-groove portion means the length of the sub-groove portion in a direction orthogonal to a direction in which the sub-groove portion extends in the object portion.

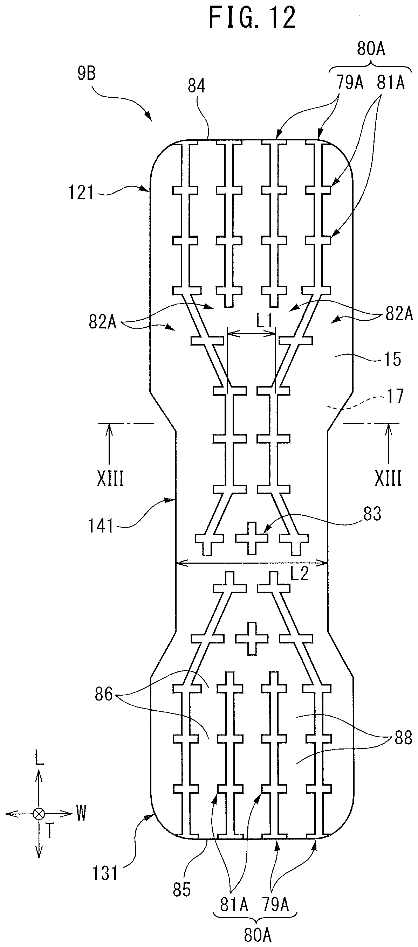

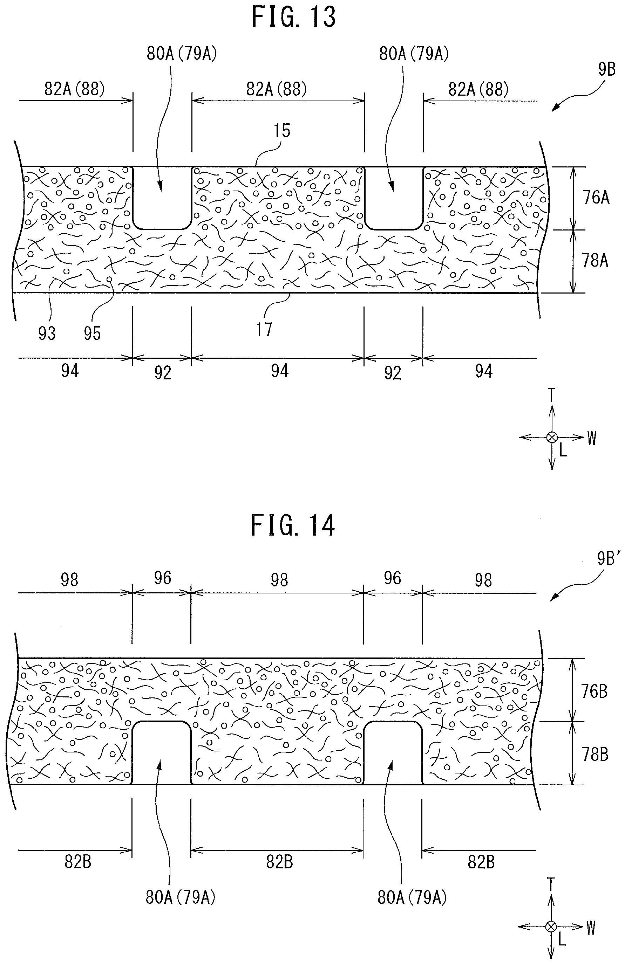

An absorbent core 9B shown in FIG. 12 has the same outer shape as that of the absorbent body 7A shown in FIG. 1. That is, the absorbent core 9B has a first end portion 121, a constricted portion 141, and a second end portion 131 in this order in the lengthwise direction. As shown in FIG. 13, the absorbent core 9B includes a skin side layer 76A on the skin facing surface 15 side and a non-skin side layer 78A on the non-skin facing surface 17 side. The skin side layer 76A includes a plurality of groove portions 80A that penetrate the skin side layer 76A in the thickness direction T, and a base 82A that is located between the plurality of groove portions 80A.

The groove portion 80A includes a plurality of main groove portions 79A and a plurality of sub-groove portions 81A. The plurality of main groove portions 79A are respectively recessed in the thickness direction T of the absorbent core 9B from the skin facing surface 15 toward the non-skin facing surface 17, and extend in the lengthwise direction L. In the case of the third embodiment, four main groove portions 79A are evenly arranged in the width direction W with an end 84 of the first end portion 121 and an end 85 of the second end portion 131 serving as base ends. Among the four main groove portions 79A at the first end portion 121 and the second end portion 131, two inner main groove portions have leading ends respectively towards the first end portion 12 and towards the second end portion 13 with respect to the constricted portion 141. The main groove portion 79A has a width that is preferably 0.5 to 3.0 times, more preferably 0.8 to 2.5 times, and even more preferably 1.0 to 2.0 times the thickness of the absorbent body 7A. When the width of the main groove portion 79A is within the above range, the main groove portion 79A easily maintains its water flow function after the absorbent article 1 absorbs the bodily fluid. Each of the plurality of sub-groove portions 81A has a base end that communicates with the main groove portion 79A and has a leading end at a position that extends in the width direction W. The sub-groove portion 81A extends along the width direction W from the first end portion 121 to the constricted portion 141 and from the second end portion 131 to the constricted portion 141 even in a range where the main groove portion 79A is inclined. The depth and the width of the sub-groove portion 81A are the same as the depth of the main groove portion 79A. Usually, the sub-groove portions 81A are formed on both sides of the main groove portion 79A. However, in the main groove portions 79A at both ends positioned on the outermost sides of the absorbent core 9B in the width direction W, the sub-groove portions 81A on the outer sides of the main groove portions 79A can be omitted. The base 82A has a narrow portion 86 positioned between the sub-groove portions 81A, and a wide portion 88 positioned between the main groove portions 79A.

The non-skin side layer 78A includes, as low-basis-weight portions having a small basis weight of the superabsorbent polymer particles, in the thickness direction T, a plurality of groove portion corresponding portions 92 at positions that overlap the plurality of groove portions 80A, and a plurality of base corresponding portions 94 at positions that overlap the plurality of bases 82A. The non-skin facing surface 17 of the non-skin side layer 78A is flat. The plurality of base potions 82A, the plurality of groove portion corresponding portions 92, and the plurality of base corresponding portions 94 may be integrally formed.

The absorbent core 9B includes water-absorbent fibers 93 and superabsorbent polymer particles (SAP) 95, and has a function of absorbing and holding the bodily fluid discharged to the absorbent article 1. The ratio of the superabsorbent polymer particles contained in the non-skin side layer (groove portion corresponding portions 92, base corresponding portions 94) 78A to the superabsorbent polymer particles contained in the entire absorbent core 9B is 0% or more and less than 50%, preferably 0% or more and less than 30%, and more preferably 0% or more and 20% or less. The average basis weight of the water-absorbent fibers in both the skin side layer 76A and the non-skin side layer 78A is preferably 50 to 250 g/m², and more preferably 80 to 200 g/m². The average basis weight of the superabsorbent polymer particles in the non-skin side layer 78A is preferably 0 to 200 g/m², and more preferably 0 to 150 g/m², and the average basis weight of the superabsorbent polymer particles in the skin side layer 76A is preferably 100 to 500 g/m², and more preferably 150 to 400 g/m². The average density (hereinafter, referred to as "first average density") of the superabsorbent polymer particles in the non-skin side layer 78A is smaller than the average density (hereinafter, referred to as "second average density") of the superabsorbent polymer particles in the skin side layer (base 82A) 6A. The first average density is, for example, 0 to 0.15 g/cm³, 0 to 0.1 g/cm³, or 0 to 0.08 g/cm³, and the second average density is, for example, 0.03 to 0.4 g/cm³, 0.04 to 0.35 g/cm³, or 0.05 to 0.3 g/cm³.

The absorbent core 9B can be formed, for example, by depositing materials that constitute the absorbent core 9B (for example, pulp and SAP) in a mold including recessed portions having protrusions in regions corresponding to the main groove portions 79A or in regions corresponding to the main groove portions 79A and the sub-groove portions 81A. Specifically, the absorbent core can be formed according to the method described in Japanese Unexamined Patent Publication No. 2010-233839, Japanese Unexamined Patent Publication No. 2014-136126, or the like of the same applicant. Specifically, the pulp is overlaid while being air-transported into the molding recessed portion of the suction box while being pulverized into a cotton-like shape with a hammer mill. Protrusions corresponding to the groove portions are located in the molding recessed portion. After the absorbent core 9B is taken out from the molding recessed portion, the absorbent core 9B can be formed by turning the absorbent core upside down. The blending ratio of the superabsorbent polymer particles (SAP) can be adjusted by dividing the pulp supply time into the first half and the second half and changing the supply amount of the SAP. The first half of the supply time of the pulp and the SAP corresponds to the skin side layer 76A, and the second half corresponds to the non-skin side layer 78A. By increasing the supply amount of the SAP in the first half of the supply time of the pulp and the SAP and decreasing the supply amount of the SAP in the second half, the absorbent core 9B in which the groove portion corresponding portions 92 as low-basis-weight portions are formed in the non-skin side layer 78A can be prepared.

The bodily fluid that has flowed into the groove portions 80A is transported in the lengthwise direction L of the absorbent core 9B from the excrement portion through the main groove portions 79A, and is absorbed into the inside of the absorbent core 9B from the main groove portions 79A and the sub-groove portions 81A that communicate with the main groove portions 79A. Further, while the bodily fluid that has permeated into the groove portion corresponding portion 92 is diffused through the base corresponding portion 94, the bodily fluid can be sucked up to the base 82A containing a large amount of superabsorbent polymer particles and having a high density, and held in the superabsorbent polymer particles. As described above, in the absorbent article, the bodily fluid is transported to a position spaced apart from the excrement portion of the absorbent core through the plurality of groove portions. Therefore, the bodily fluid is absorbed in a larger area of the absorbent core, and a decrease in the absorption power of the absorbent core in the vicinity of the excrement portion can be suppressed. Therefore, even when the bodily fluid is repeatedly absorbed, the absorbent article 1 can maintain the absorbency, for example, absorption rate, of the entire absorbent core by repeating the above cycle.

The absorbent core 9B according to the third embodiment is enclosed by a core-wrapping sheet and used in the absorbent article. The core-wrapping sheet has an inner layer containing 50 mass% or more of cellulose fibers and an outer layer containing 50 mass% or more of thermoplastic synthetic fibers. Since the absorbent article includes the absorbent core having the groove portions and the inner layer having an excellent diffusivity, the liquid absorbency can be maintained at a higher level. The absorbent article has the inner layer, which makes it possible to enhance the deformation resistance. When the absorbent article having the inner layer was put on a doll in a state where artificial urine was absorbed and a certain movement was performed, it was confirmed that the amount of deformation was small compared with the conventional absorbent article having no inner layer.

The inner layer may have a plurality of open holes that penetrate the inner layer in the thickness direction. The outer layer has a plurality of first grooves recessed in the thickness direction T on the first surface and a plurality of second grooves recessed in the thickness direction T on the second surface 31. Each of the plurality of first grooves and each of the plurality of second grooves are alternately arranged with predetermined spaces in the direction parallel to the width direction W. The inner layer may be colored in a different color from the color of the absorbent core. The colored inner layer can be visually recognized through the low-basis-weight portion of the absorbent body. In the absorbent body that does not absorb the bodily fluid, the position of the groove portion corresponding portion 92 can be confirmed with the color of the inner layer that has transmitted through the groove portion corresponding portion 92 which is a low-basis-weight portion. Therefore, in a dry state, the user can use the low-basis-weight portion as an index for position alignment. Since the absorbent body that has absorbed the bodily fluid is wetted by the absorbed bodily fluid, the inner layer that has transmitted through the low-basis-weight portion is less likely to be visually recognized. Therefore, in a wet state, the user can visually recognize the absorption state of the bodily fluid. Here, the coloring agent used for coloring the inner layer is not particularly limited, and any coloring agent known in the art (for example, a pigment, a dye, or the like) can be employed. Further, there is no particular limitation on the coloring means using this coloring agent, and the inner layer may be colored by, for example, applying the coloring agent or performing printing with the coloring agent, or causing the coloring agent to be contained in the constituent component of the inner layer.

The absorbent core may have groove portions in the non-skin side layer. An absorbent core 9B' shown in FIG. 14 includes, in a non-skin side layer 78B, the plurality of groove portions 80A that penetrate the non-skin side layer 78B in the thickness direction T, and a base 82B serving as a low-basis-weight portion and located between the plurality of groove portions 80A. A skin side layer 76B includes groove portion corresponding portions 96 as low-basis-weight portions at a position corresponding to the groove portions 80A, and base corresponding portions 98. The average density of the superabsorbent polymer particles in the base 82B is preferably 0 to 0.15 g/cm³, and more preferably 0 to 0.1 g/cm³. The average density of the superabsorbent polymer particles in the groove portion corresponding portion 96 is preferably 0 to 0.15 g/cm³, and more preferably 0 to 0.1 g/cm³. The average density of the superabsorbent polymer particles in the base corresponding portion 98 is preferably 0.1 to 0.3 g/cm³, and more preferably 0.1 to 0.2 g/cm³. The absorbent core 9B' can be obtained by preparing the absorbent core 9B' in the same method as used for the absorbent core 9B, taking out the absorbent core 9B' from the molding recessed portion, and then keeping the absorbent core 9B as it is without turning the absorbent core upside down. The third embodiment can be combined with the first embodiment or the second embodiment, and by combining these embodiment, the same effect as that of the first embodiment can be obtained.

### 4. Evaluation

The absorbent articles corresponding to the above embodiments were actually prepared and evaluated. Hereinafter, the present invention will be described by showing examples, but the present invention is not limited to these examples.

### (1) Sample

### [Lower Core-Wrapping Sheet A]

A lower core-wrapping sheet A was prepared by processing a hydrophilic polypropylene spunbond (PPSB) nonwoven fabric (basis weight: 15 g/m²) as a raw material using the manufacturing apparatus 41 shown in FIGS. 5 and 11. Specifically, the polypropylene spunbond nonwoven fabric was preheated with the pair of heating rolls having an outer circumferential surface temperature of 90°C to 95°C, and then shaped with the pair of stretching rolls. The transport rate of the nonwoven fabric was 150 m/min. The shaped nonwoven fabric had a plurality of first grooves and a plurality of second grooves, and had a plurality of recessed portions corresponding to the recessed portions shown in FIG. 9 on the surface that came into contact with the stretching rolls. The plurality of first grooves and the plurality of second grooves extended in a manner of continuing from one end edge to the other end edge of the core-wrapping sheet. In the shaped nonwoven fabric, the side having the plurality of recessed portions corresponds to the core facing surface side of the sheet in the absorbent article. The shaped nonwoven fabric was cut to a size of 355 mm × 140 mm to prepare the lower core-wrapping sheet A. In a case of determination by electron microscope observation, on the upper surface side of the lower core-wrapping sheet A, the height from the bottom portion of the continuous first groove to the apex portion of the continuous first ridge was 1.0 mm, and the space between the apex portions of adjacent first ridges was 1.0 mm. The recessed portion had a substantially rectangular parallelepiped space, and the depth of the recessed portion was approximately 0.2 mm.

### [Lower Core-Wrapping Sheet B]

A tissue (basis weight: 16 g/m², 355 mm × 140 mm) formed of wood pulp as cellulose fibers was used as an inner layer, and the lower core-wrapping sheet A overlapped the inner layer as an outer layer to prepare a lower core-wrapping sheet B. The tissue of the lower core-wrapping sheet B is not shaped.

### [Lower Core-Wrapping Sheet C]

A tissue (basis weight: 16 g/m²) formed of wood pulp as cellulose fibers was used as the inner layer continuous body 116, and a hydrophilic polypropylene spunbond (PPSB) nonwoven fabric (basis weight: 15 g/m²) was processed using the manufacturing apparatus 41 shown in FIGS. 5 and 11 as the outer layer continuous body 118. Specific conditions are the same as those as those for the lower core-wrapping sheet A. The shaped two-ply nonwoven fabric was held in a state where the tissue and the polypropylene spunbond nonwoven fabric overlapped each other, and had a plurality of core facing grooves and a plurality of non-core facing grooves (second grooves). In the core facing groove, the inner layer groove of the tissue and the first groove of the polypropylene spunbond nonwoven fabric overlapped each other. The inner layer continuous body and the outer layer continuous body had a plurality of open holes corresponding to the open holes shown in FIG. 9 and a plurality of recessed portions corresponding to the recessed portions shown in FIG. 9. The shaped nonwoven fabric was cut into a size of 355 mm × 100 mm to prepare a lower core-wrapping sheet C.

### [Lower Core-Wrapping Sheet D]

In the shaped two-ply nonwoven fabric obtained when the lower core-wrapping sheet C was prepared, the tissue was peeled off from the polypropylene spunbond nonwoven fabric. The tissue was cut to a size of 355 mm × 100 mm, and the polypropylene spunbond nonwoven fabric was cut to a size of 355 mm × 140 mm. The tissue overlapped the polypropylene spunbond nonwoven fabric again so that the centers thereof in the width direction coincided with each other, and a lower core-wrapping sheet D was prepared. Since the tissue was once peeled off from the polypropylene spunbond nonwoven fabric, the positions of the inner layer groove of the tissue and the first groove of the polypropylene spunbond nonwoven fabric were deviated and did not overlap each other.

### [Upper Core-Wrapping Sheet]

A hydrophilic polypropylene spunbond (PPSB) nonwoven fabric (basis weight: 15 g/m²) was cut to a size of 355 mm × 100 mm or 355 mm × 140 mm to prepare an upper core-wrapping sheet D having two sizes.

As shown in FIG. 2, in a case where the side surface of the absorbent core was covered with the lower core-wrapping sheet from the non-skin facing surface, the upper core-wrapping sheet having a size of 355 mm × 100 mm was used. The skin facing surface was covered with the upper core-wrapping sheet, and the lower core-wrapping sheet and the upper core-wrapping sheet were joined to each other on the skin facing surface. In Table 1 below, the absorbent body joined to the skin facing surface is denoted as "Upper surface side" with respect to "Joining position of upper and lower core-wrapping sheets".

As shown in FIG. 7A, in a case where the side surfaces of the absorbent core were covered with the upper core-wrapping sheet from the skin facing surface, the upper core-wrapping sheet having a size of 355 mm × 140 mm was used. The non-skin facing surface was covered with the lower core-wrapping sheet, and the upper core-wrapping sheet and the lower core-wrapping sheet were joined to each other on the non-skin facing surface. In Table 1 below, the absorbent body joined to the non-skin facing surface is denoted as "Lower surface side" with respect to "Joining position of upper and lower core-wrapping sheets".

### [Absorbent Core A]

Water-absorbent fibers (pulp) were pulverized into a cotton-like shape in a hammer mill so as to have the basis weight described in Table 1, and were overlaid while being air-transported into the molding recessed portion having a size of 335 mm × 100 mm (the lengthwise direction × the width direction) of the suction box. The water-absorbent fibers were taken out from the molding recessed portion and then turned upside down to form an absorbent core. The blending ratio of the superabsorbent polymer particles (SAP) was adjusted by dividing the pulp supply time into the first half and the second half and changing the supply amount of the SAP. The first half of the supply time of the pulp and the SAP corresponds to the skin side layer, and the second half corresponds to the non-skin side layer. By setting supply amount of the SAP in the first half and the second half at the same amount, an absorbent core A with a uniform blend was obtained.

### [Absorbent Core B]

An absorbent core B having groove portions on the skin facing surface was prepared by the same procedure as that of the absorbent core A except that the molding recessed portion having protrusions corresponding to the groove portions was used. A blending ratio of the superabsorbent polymer particles (SAP) of 100/0 in the skin side layer and the non-skin side layer was prepared by supplying the entire supply amount of the SAP in the first half and overlaying the pulp in the second half without supplying the SAP. In this manner, an absorbent core B having groove portions corresponding to the groove portions shown in FIG. 12 in the skin side layer was obtained. The width length of the main groove portion was 4 mm, the width length of the sub-groove portion was 6 mm, and the lengthwise length of the sub-groove portion was 4 mm.

### [Absorbent Article]

An absorbent body was prepared by covering the absorbent core with the lower core-wrapping sheet arranged on the non-skin facing surface and the upper core-wrapping sheet arranged on the skin facing surface. The thickness of the absorbent core was adjusted by pressing the absorbent body with a hydraulic press machine. Thereafter, a liquid-permeable sheet (air-through nonwoven fabric) was attached to the layer side on the skin side of each absorbent body, and a liquid-impermeable sheet (polyethylene film) was attached to the layer side on the non-skin side. Simple absorbent articles of Examples 1 to 5, Comparative Example 1, and Reference Example 1 were thereby prepared.

### (2) Test Method

The obtained absorbent article samples were evaluated by the following test method, and the evaluation results are shown in Table 1 below together with the configurations of the absorbent bodies.

### [Absorbency Test]

(1) A cross was marked at a central position A of the absorbent article in the lengthwise direction, and a cross was further marked at a position B 50 mm on the stomach-side of the absorbent article in the lengthwise direction. The absorbent article is set in a U-shaped instrument having a substantially U-shaped side view. It should be noted that the absorbent article is set so that the central position of the absorbent body in the lengthwise direction matches the central part of the U-shaped instrument (the position at which the height is the lowest).

### <First Cycle>

(2) 80 mL of artificial urine (first time) is injected from a burette at a rate of 80 mL/10 sec to the position B of the absorbent body.

(3) The time from the start of injection of the artificial urine of the first time until the artificial urine within the U-shaped instrument disappears is recorded as the absorption time (80 mL).

### <Second Cycle>

(4) After 10 minutes from the start of the injection of the artificial urine of the first time, 80 mL of artificial urine (second time) is injected into the position B of the absorbent body from a burette at a rate of 80 mL/10 sec.

(5) The time from the start of injection of the artificial urine of the second time until the artificial urine within the U-shaped instrument disappears is recorded as the absorption time (160 mL).

### <Third Cycle>

(6) The operations of (4) and (5) are repeated and the absorption time (240 mL) is measured.

It should be noted that the artificial urine was prepared by dissolving 200 g of urea, 80 g of sodium chloride, 8 g of magnesium sulfate, 3 g of calcium chloride, and approximately 1 g of a dye (blue No.1) in 10 L of ion exchange water.

### [Table 1]

**Table 1**

| | | Examp le 1 | Examp le 2 | Examp le 3 | Examp le 4 | Examp le 5 | Comparati ve Example 1 | Referen ce exampl e 1 |
|---|---|---|---|---|---|---|---|---|
| Configurat ion of absorbent core | Absorbent core | A | A | B | B | B | A | A |
| | Pulp basis weight (g/m²) | 165 | 165 | 165 | 165 | 165 | 165 | 165 |
| | SAP basis weight (g/m²) | 317 | 317 | 317 | 317 | 317 | 317 | 317 |
| | SAP distributio n (skin side layer/non-skin side layer) | Uniform | Uniform | 100/0 | 100/0 | 100/0 | Uniform | Uniform |
| | Presence or absence of groove portion | Absent | Absent | Presen t | Presen t | Presen t | Absent | Absent |
| Configurat ion of lower core-wrapping sheet | Lower corewrapping sheet | D | C | D | C | B | A | B |
| | Presence or absence of inner layer | Presen t | Presen t | Presen t | Presen t | Presen t | Absent | Present |
| | Presence or absence of inner layer/open hole | Presen t | Presen t | Presen t | Presen t | Absent | - | Absent |
| | Joining position of upper and lower core-wrapping sheets | Upper surface side | Lower surface side | Upper surface side | Lower surface side | Upper surface side | Upper surface side | Upper surface side |
| Absor tion time (sec) | 80 ml | 23 | 22 | 17 | 19 | 20 | 22 | 22 |
| | 160 ml | 35 | 34 | 19 | 22 | 23 | 35 | 40 |
| | 240 ml | 77 | 75 | 34 | 36 | 55 | 122 | 111 |

In Examples 1 to 5, the core-wrapping sheet has the inner layer and the outer layer, the inner layer contains 50 mass% or more of cellulose fibers, the outer layer contains 50 mass% or more of thermoplastic synthetic fibers, and has the plurality of first grooves recessed in the thickness direction on the first surface and the plurality of second grooves recessed in the thickness direction on the second surface, and each of the plurality of first grooves and each of the plurality of second grooves are alternately arranged with predetermined spaces. In comparison of Examples 1 to 5 with Comparative Example 1, it was found that since the core-wrapping sheet had the inner layer, the absorption time in the third cycle was shortened. The inner layer of each of Examples 1 to 4 has a plurality of open holes, but the inner layer of each of Example 5 and Reference Example 1 does not have open holes. In comparison of Examples 1 and 2 with Reference Example 1, it was confirmed that since the inner layer had open holes, the absorption time in the third cycle was shortened. The absorbent core of each of Examples 1 and 2 does not have groove portions, but the absorbent core of each of Examples 3 and 4 has groove portions. In comparison of Examples 1 and 2 with Examples 3 and 4, it was confirmed that since the absorbent core had groove portions, the absorption time in the third cycle was shortened. The inner layer of Example 5 does not have open holes, but the absorbent core has groove portions. In Example 5, as compared with Reference Example 1, it was confirmed that since the absorbent core had groove portions, the absorption time was significantly shortened.

### REFERENCE SIGNS LIST

1: absorbent article
3: top sheet
5: Back sheet
7A to 7D: absorbent body
9A, 9B: absorbent core
11A to 11E: core-wrapping sheet
15: skin facing surface
16A, 16B: inner layer
17: non-skin facing surface
18A, 18D: outer layer
21: core facing surface
23: non-core facing surface
25: inner layer groove
27: open hole
29: first surface
31: second surface
33: first groove
35: second groove
76A, 76B: skin side layer
78A and 78B: non-skin side layer
80A: groove portion
82A: base

## Claims

1. An absorbent article comprising:
a liquid-permeable top sheet;
a liquid-impermeable back sheet; and
an absorbent body that is arranged between the top sheet and the back sheet, wherein
the absorbent body includes
an absorbent core having a skin facing surface and a non-skin facing surface on a side opposite to the skin facing surface, and having a lengthwise direction, a width direction, and a thickness direction, and
a core-wrapping sheet that is arranged at least one side of the skin facing surface and the non-skin facing surface,
the core-wrapping sheet has an inner layer and an outer layer,
the inner layer contains 50 mass% or more of cellulose fibers, and has a core facing surface in contact with the absorbent core, a non-core facing surface on a side opposite to the core facing surface, and a plurality of open holes penetrating the inner layer from the core facing surface to the non-core facing surface,
the outer layer contains 50 mass% or more of thermoplastic synthetic fibers and has a first surface in contact with the non-core facing surface, a second surface on a side opposite to the first surface, a plurality of first grooves recessed in the thickness direction on the first surface, and a plurality of second grooves recessed in the thickness direction on the second surface, and
each of the plurality of first grooves and each of the plurality of second grooves are alternately arranged with predetermined spaces.

2. The absorbent article according to Claim 1, wherein
the non-core facing surface and the first surface are not joined to each other.

3. The absorbent article according to Claim 1 or 2, wherein
each of the plurality of first grooves and each of the plurality of second grooves extend in a direction parallel to the lengthwise direction, and are alternately arranged with predetermined spaces in a direction parallel to the width direction.

4. The absorbent article according to any one of Claims 1 to 3, wherein
the inner layer has a plurality of inner layer grooves recessed in the thickness direction on the core facing surface.

5. The absorbent article according to Claim 4, wherein
each of the plurality of inner layer grooves overlaps each of the plurality of first grooves.

6. The absorbent article according to any one of Claims 1 to 5, wherein
the inner layer is formed of a tissue, and the outer layer is formed of a spunbond nonwoven fabric.

7. The absorbent article according to Claim 6, wherein
the inner layer and the absorbent core are joined with a hot-melt adhesive.

8. The absorbent article according to any one of Claims 1 to 7, wherein
the outer layer and the absorbent core are joined through the plurality of open holes.

9. The absorbent article according to any one of Claims 1 to 8, wherein
the absorbent core has a low-basis-weight portion in which a basis weight of an absorbent material is smaller than a surrounding region, and
the inner layer is colored in a different color from a color of the absorbent core.

10. The absorbent article according to any one of Claims 1 to 9, wherein
the core-wrapping sheet is arranged on a non-skin facing surface side.

11. The absorbent article according to Claim 10, wherein
the inner layer has a plurality of inner layer grooves recessed in the thickness direction on the core facing surface, and each of the plurality of open holes is formed in each of the plurality of inner layer grooves.

12. The absorbent article according to any one of Claims 1 to 11, wherein
the absorbent core includes a skin side layer including the skin facing surface, and a non-skin side layer including the non-skin facing surface,
the skin side layer or the non-skin side layer includes
a plurality of groove portions including a plurality of main groove portions that penetrate in the thickness direction, and
a plurality of bases, and
each of the plurality of groove portions and each of the plurality of bases are alternately arranged.

13. An absorbent article comprising:
a liquid-permeable top sheet;
a liquid-impermeable back sheet; and
an absorbent body that is arranged between the top sheet and the back sheet, wherein
the absorbent body includes
an absorbent core having a skin facing surface and a non-skin facing surface on a side opposite to the skin facing surface, and having a lengthwise direction, a width direction, and a thickness direction, and
a core-wrapping sheet that is arranged at least one side of the skin facing surface and the non-skin facing surface,
the absorbent core includes a skin side layer including the skin facing surface, and a non-skin side layer including the non-skin facing surface, the skin side layer or the non-skin side layer includes a plurality of groove portions including a plurality of main groove portions that penetrate in the thickness direction, and a plurality of bases, and each of the plurality of groove portions and each of the plurality of bases are alternately arranged,
the core-wrapping sheet has an inner layer and an outer layer,
the inner layer contains 50 mass% or more of cellulose fibers, and has a core facing surface in contact with the absorbent core, and a non-core facing surface on a side opposite to the core facing surface,
the outer layer contains 50 mass% or more of thermoplastic synthetic fibers and has a first surface in contact with the non-core facing surface, a second surface on a side opposite to the first surface, a plurality of first grooves recessed in the thickness direction on the first surface, and a plurality of second grooves recessed in the thickness direction on the second surface, and
each of the plurality of first grooves and each of the plurality of second grooves are alternately arranged with predetermined spaces.

14. A method of manufacturing a core-wrapping sheet for an absorbent body including an absorbent core and an absorbent core-wrapping sheet,
the core-wrapping sheet including
an outer layer containing 50 mass% or more of thermoplastic synthetic fibers, and having a first surface and a second surface on a side opposite to the first surface, and
an inner layer containing 50 mass% or more of cellulose fibers, and having a core facing surface and a non-core facing surface on a side opposite to the core facing surface,
the method comprising:
a step of preheating an outer layer continuous body formed of the outer layer; and
a step of transporting the preheated outer layer continuous body and an inner layer continuous body formed of the inner layer between a pair of shaping members including a first shaping member and a second shaping member in a state where the first surface and the non-core facing surface are in contact with each other, and forming a plurality of core facing grooves recessed in a thickness direction on the core facing surface, a plurality of non-core facing grooves recessed in the thickness direction on the second surface, and a plurality of open holes penetrating the inner layer continuous body from the core facing surface to the non-core facing surface in the inner layer continuous body.
